(19) Europäisches Patentamt
European Patent Office
Office européen des brevets

(11) EP 1 519 946 B1

(12) FASCICULE DE BREVET EUROPEEN

(45) Date de publication et mention de la délivrance du brevet:
**28.04.2010 Bulletin 2010/17**

(21) Numéro de dépôt: **03761628.1**

(22) Date de dépôt: **23.06.2003**

(51) Int Cl.:
***C07K 14/01*** *(2006.01)*

(86) Numéro de dépôt international:
**PCT/FR2003/001926**

(87) Numéro de publication internationale:
**WO 2004/003012 (08.01.2004 Gazette 2004/02)**

# (54) NOUVEAUX VARIANTS DU POLYPEPTIDE PAPM DE BACTERIES DU GENRE STREPTOMYCES.

NEUE VARIANTEN DES PAPM-POLYPEPTIDS VON BAKTERIEN DER GATTUNG STREPTOMYCES

NOVEL VARIANTS OF THE PAPM POLYPEPTIDE OF BACTERIA OF GENUS STREPTOMYCES

(84) Etats contractants désignés:
**AT BE BG CH CY CZ DE DK EE ES FI FR GB GR HU IE IT LI LU MC NL PT RO SE SI SK TR**

(30) Priorité: **28.06.2002 FR 0208057**

(43) Date de publication de la demande:
**06.04.2005 Bulletin 2005/14**

(73) Titulaire: **Aventis Pharma S.A.**
**92160 Antony (FR)**

(72) Inventeurs:
- **BAMAS-JACQUES, Nathalie**
  **F-75005 Paris (FR)**
- **THIBAUT, Denis**
  **F-75013 Paris (FR)**
- **FAMECHON, Alain**
  **F-91510 Janville sur Juine (FR)**

(74) Mandataire: **Dernoncour, Roxane**
**Aventis Pharma S.A.**
**Direction des Brevets,**
**Tri LEO/144**
**20, avenue Raymond Aron**
**92165 Antony Cedex (FR)**

(56) Documents cités:
**WO-A-96/05219 US-B1- 6 352 839**

- **BLANC V ET AL: "IDENTIFICATION AND ANALYSIS OF GENES FROM STREPTOMYCES PRISTINAESPIRALIS ENCODING ENZYMES INVOLVED IN THE BIOSYNTHESIS OF THE 4-DIMETHYLAMINO-L-PHENYLALANINE PRECURSOR OF PRISTINAMYCIN I" MOLECULAR MICROBIOLOGY, BLACKWELL SCIENTIFIC, OXFORD, GB, vol. 23, no. 2, 1997, pages 191-202, XP002934995 ISSN: 0950-382X**

Il est rappelé que: Dans un délai de neuf mois à compter de la publication de la mention de la délivrance du brevet européen au Bulletin européen des brevets, toute personne peut faire opposition à ce brevet auprès de l'Office européen des brevets, conformément au règlement d'exécution. L'opposition n'est réputée formée qu'après le paiement de la taxe d'opposition. (Art. 99(1) Convention sur le brevet européen).

EP 1 519 946 B1

Printed by Jouve, 75001 PARIS (FR)

## Description

La présente invention concerne de nouveaux variants du polypeptide PapM de bactéries du genre Streptomyces possédant une sélectivité de substrat et/ou une efficacité améliorée par rapport au polypeptide de type sauvage. Elle se rapporte également aux acides nucléiques codant pour ces variants, aux microorganismes incorporant ces acides nucléiques et leur utilisation pour la production de composantes B des Streptogramines.

La pristinamycine est un antibiotique qui appartient à la famille des streptogramines à laquelle appartiennent aussi la virginiamycine et la mikamycine. Les streptogramines forment un petit groupe homogène et original d'antibiotiques constitués d'une association de deux types de molécules chimiquement très différentes : les streptogramines A sont des macrolactones polyinsaturées et les streptogramines B sont des depsipeptides (Cocito C. G. (1979) Microbiol. Rev., 43 : 145-198 ) (Cocito C. G. (1983) In Antibiotics, 6 : (Ed. F. E. Hahn), 296-332.). Dans le cas de la pristinamycine, les composés A et B sont appelées respectivement pristinamycines II (PII) et pristinamycines I (PI) et leurs structures sont représentées sur les figures 1 et 2.

Les composés A et B ont une activité antibactérienne synergique et bactéricide particulièrement dirigée contre les bactéries Gram-positive telles que les Staphylocoques et les Entérocoques (Cocito, 1979). Ces composés agissent en se fixant à la sous-unité 50S des ribosomes des cellules cibles ce qui provoque une inhibition de la synthèse protéique (Cocito, 1979) ; (Di Giambattista M., Chinali G. et Cocito C. G. (1989) J. Antim. Chemother., 24 : 485-507 ).

Les Streptogramines sont essentiellement produites par des Actinomycètes. Plus particulièrement, la pristinamycine est produite par la bactérie filamenteuse *Streptomyces pristinaespiralis* sous la forme d'un mélange naturel constitué de 30 % de PI et de 70% de PII (Blanc et al., (1995), J. Bacteriol. 177 (18) : 5206-14). Pour chaque type de molécules, plusieurs isoformes sont coproduites comme il est fréquemment observé pour des métabolites naturels.

Dans le cas des pristinamycines PI (composantes B des pristinamycines), la forme majoritairement produite est la PIA qui est constituée des 7 résidus suivants reliés entre eux par des liaisons amidiques et une liaison ester (Figure 2) : acide 3-hydroxypicolinique, L-thréonine, acide D-aminobutyrique, L-proline, 4-diméthylamino-L-phénylalanine, acide 4-oxo-L-pipécolique et L-phénylglycine. Plusieurs isoformes naturelles sont aussi coproduites avec la PIA et correspondent à des modifications structurales portant principalement sur les résidus suivants : acide D-aminobutyrique, 4-diméthylamino-L-phénylalanine (L-DMPAPA) et acide 4-oxo-L-pipécolique (Thibaut et al., (1997) J. Bacteriol. 179(3) :697-704). Ces formes minoritaires sont synthétisées avec des pourcentages variables selon les souches de *S. pristinaespiralis* qui restent globalement de l'ordre de quelques pourcents, la PIA représentant de 90% à 95% des PI produites. On remarque en particulier que les différentes souches de *S. pristinaespiralis* synthétisent environ 5% de PIB qui se différencie de la PIA par la présence d'un 4-méthylamino-L-phénylalanine (L-MMPAPA) en position 4 du macrocycle (figure 2). Bien que minoritaire, la PIB s'est révélé être une molécule particulièrement intéressante pour ses différentes propriétés pharmacologiques (WO96/05219).

Un autre analogue de la PIA, la PINH2 qui se différencie par la présence d'une 4-amino-L-phénylalanine en position 4 (voir figure 2) présente un intérêt pour élaborer des produits originaux d'hémisynthèse. La PINH2 n'a pu être obtenue jusqu'à présent que dans un procédé dans lequel la 4-amino-L-phénylalanine est ajoutée dans le milieu de culture de *S. pristinaespiralis* (WO 96/01901).

Plusieurs approches ont été envisagées pour améliorer la production de Streptogramines, une première approche vise à modifier la proportion totale de composantes A (pristinamycines II) produites par rapport à la production de composantes B (pristinamycines I), une autre approche vise à modifier la proportion des différentes isoformes présentes au sein de chaque composantes A ou B. Toutefois aucune approche n'a permis à ce jour de produire de manière satisfaisante la pristinamycine PIB qui reste une forme minoritaire des composantes B (pristinamycines I) de la pristinamycine.

Une première approche décrite dans la demande WO93/20182 consiste dans la sélection de microorganimes capables de produires spécifiquement les composantes A ou les composantes B des streptogramines. Ces microorganismes sont obtenus par des techniques classiques de sélection comportant une première étape facultative de mutagénèse sur un microorganisme producteur de streptogramines et une deuxième étape d'identification de microorganismes produisant de manière sélective l'une ou l'autre des composantes A ou B des streptogramines. Cette technique permet notamment d'obtenir des souches de *S. pristinaespiralis* produisant sélectivement des Pristinamycines PII (composante A) ou des pristinamycines PI (composante B). Cependant ce document ne décrit pas de souches produisant sélectivement des pristinamycines PI avec une proportion d'isoformes modifiée en faveur de la forme PIB par rapport à la PIA qui reste toujours majoritaire.

La demande WO 94/08014 décrit l'isolement et l'identification de gènes codant pour des enzymes impliquées dans la voie de biosynthèse des composantes A et dans la voie de biosynthèse des composantes B des streptogramines. Elle décrit également l'expression de ces gènes dans le but d'augmenter les taux de production des composantes A ou B et l'utilisation de ces gènes pour la construction de mutants bloqués à différentes étapes des voies de biosynthèse des composantes A ou B et conduisant ainsi à l'accumulation de certains intermédiaires de ces voies de biosynthèse. Plus particulièrement la demande WO94/08014 décrit l'isolement et la caractérisation de douze gènes snaA, snaB, snaC, snaD, papA, papM, samS, snbA, snbC, snbD, snbE et snbR isolés à partir d'une banque d'ADN génomique de *S. pristinaespiralis* et met en évidence l'implication des gènes snaA, snaB, snaC, snaD dans la voie de biosynthèse des composantes A et celle des gènes papA, papM, samS, snbA, snbC, snbD, snbE et snbR dans la voie de biosynthèse des composantes B.

- Ainsi l'inactivation du gène snaA dans une souche de *S. pristinaespiralis* permet d'obtenir une souche ne produisant plus de PIIA (une des isoformes des composantes A des pristinamycines) mais produisant uniquement de la PIIB (autre isoforme des composantes A des pristinamycines) en quantité équivalente à la somme des PIIA et PIIB produites par la souche contrôle portant la forme sauvage du gène snaA. La production de PI (ensembles des isoformes des composantes B des pristinamycine) reste identique dans les souches inactivées pour le gène snaA et dans dans la souche contrôle.

- L'inactivation du gène samS conduit à l'obtention d'une souche qui produit 35 % moins de PIA (une des isoformes des composantes B des pristinamycines) et environ 10 fois plus de PIB (autre isoforme des composantes B des pristinamycines) par rapport à la souche contrôle ; le taux de PIB atteint alors 20 % de l'ensemble des pristinamycines du type I (PI) totales produites. Bien que la production de PIB soit améliorée dans ces souches, la proportion de PIB n'est pas majoritaire et reste inférieure à celle de PIA.

- L'inactivation du gène papA ou du gène snbA conduit à l'obtention de souches qui ne produisent que des pristinamycines de type PII (composantes A) et qui ne produisent plus de pristinamycines de type PI (composantes B).

- L'inactivation du gène snaD permet d'obtenir des souches qui, à l'inverse, ne produisent que des pristinamycines de type PI et qui ne produisent plus de pristinamycines de type PII.

La demande WO 96/01901 décrit un procédé original de préparation de nouveaux composés apparentés aux composantes B des streptogramines mais différents des composantes B produites naturellement par les microorganismes producteurs de streptogramines. Selon ce procédé, on utilise une souche de microorganisme mutée de manière à altérer la biosynthèse des précurseurs des composantes B des streptogramines. A titre de souche mutante, on peut utiliser notamment des souches de *S. pristinaespiralis* dont la voie de biosynthèse des précurseurs des composantes B est altérée par disruption des gènes papA ou pipA ou hpaA. Ces souches ne produisent plus de pristinamycines PI (composantes B) et produisent des pristinamycines PII (composantes A). La souche mutante est cultivée dans un milieu complémenté par un précurseur original, différent du précurseur dont la synthèse est altérée. L'incorporation de précurseurs originaux au sein du depsipeptide et en lieu et place des précurseurs naturels conduit à l'obtention de nouveaux composés apparentés aux composantes B des streptogramines et qui présentent des propriétés thérapeutiques intéressantes.

L'approche mentionnée ci-dessus (WO96/01901) permis d'obtenir des souches de microorganismes produisant de façon majoritaire, au sein des composantes B, des isoformes naturellement produites sous forme minoritaire. Cependant elle nécessite de supplémenter le milieu de culture avec des précurseur spécifiques non présents à l'état naturel dans les souches de microorganismes.

Les études fondamentales biochimiques et génétiques des voies de biosynthèse de la pristinamycine ont permis d'élucider l'origine des nombreuses isoformes observées pour les PI. Plus particulièrement il a été montré que la PIB provenait de l'incorporation dans le macrocycle par la peptide synthétase SnbDE du L-MMPAPA, un intermédiaire de biosynthèse du L-DMPAPA qui est incorporé au macrocycle de la PIA (Blanc et al., Mol. Microbiol (1997), 23(2) : 191-202) ; Thibaut et al, J. Bacteriol. (1997), 179 (3) : 697-704). Il a été identifié 4 gènes impliqués dans la biosynthèse du L-MMPAPA et L-DMPAPA à partir de chorismate papA, papB, papC et papM (Blanc et al., 1997).

Le gène papM code pour une protéine catalysant les deux réactions de méthylation successives qui permettent de produire le L-DMPAPA à partir de la 4-amino-L-phénylalanine visa le L-MMPAPA selon le schéma décrit dans la figure 3. Ces deux réactions de méthylation sont catalysées par PapM avec des paramètres cinétiques (constantes d'affinité et vitesses de réaction ) très similaires (Blanc et al., 1997). La séquence du gène papM de *S. pristinaespiralis* et celle du polypeptide correspondant (4-amino-L-phénylalanine (phényl N)-méthyltransférase) sont présentées dans la séquence SEQ ID N°1.

La présente invention repose sur la découverte qu'il est possible d'améliorer la production de certaines isoformes minoritaires des composantes B des streptogramines en modifiant l'activité enzymatique du produit du gène papM.

Plus particulièrement, l'invention repose sur l'identification de nouveaux variants du polypeptide PapM possédant une sélectivité de substrat et/ou une efficacité améliorée par rapport au polypeptide de type sauvage. Les inventeurs ont en effet montré qu'il est possible de mutagéniser le gène papM de telle façon que la protéine correspondante ne soit plus capable de catalyser la deuxième réaction de méthylation tout en conservant une activité suffisante pour la première réaction de méthylation, ou, tout du moins, de telle façon que la deuxième réaction de méthylation soit fortement inhibée comparativement à la première réaction.

La présence de telles enzymes dans des microorganismes producteurs de Streptogramines tels que des souches de *S. pristinaespiralis* conduit à l'accumulation de L-MMPAPA et donc à la production préférentielle de PIB (figure 3). Réciproquement, il est également possible de mutagéniser le gène papM de telle façon que la protéine correspondante catalyse la deuxième réaction de méthylation avec une efficacité supérieure à celle de la première réaction de méthylation , la présence de telles enzymes dans des souches de *S. pristinaespiralis* doit conduire à l'accumulation de L-DMPAPA et donc à la production accrue de PIA.

L'invention repose également sur la découverte que les microorganimes produisant une forme inactive du polypeptide PapM ou les mutants n'exprimant plus le gène papM permettent l'accumulation de composantes B des streptogramines que les microorganismes sauvages sont capables de synthétiser naturellement mais dans des quantités trop faibles pour être isolées. Ainsi la présente invention décrit la construction de souches recombinantes de *S. pristinaespiralis* produisant principalement un composé de la famille des PI, appelé PINH2, que de nombreuses souches sauvages de *S. pristinaespiralis* ne sont pas capables de synthétiser et qui correspond à l'incorporation d'une 4-ammo-L-phénylalanine en position 4 du macrocycle (figure 2).

Un premier objet de l'invention concerne un variant du polypeptide PapM caractérisé en ce qu'il présente une efficacité de méthylation vis à vis des substrats de méthylation différente (augmentée ou réduite) par rapport à l'efficacité de méthylation du polypeptide PapM sauvage vis à vis de ces mêmes substrats.

Le variant du polypeptide PapM caractérisé en ce qu'il présente une efficacité de méthylation 1 défie par le rapport de $Kcat_1$ / $Km_1$ vis à vis du substrat de méthylation 1 et/ou une efficacité de méthylation 2 définie par le rapport $Kcat_2$ / $Km_2$ vis à vis du substrat de méthylation 2 différente du polypeptide PapM sauvage. De manière préférée, le variant de PapM selon l'invention présente une efficacité de méthylation 1 de L-PAPA en L-MMPAPA définie par le rapport $Kca_{PAPA}$ / $Km_{PAPA}$ et/ou l'efficacité de méthylation 2 du L-MMPAPA en L-DMPAPA définie par le rapport $Kcat_{MMPAPA}$ / $Km_{MMPAPA}$ différente de l'efficacité de méthylation du polypeptide PapM présenté en SEQ ID N°1.

Selon un premier mode de réalisation, le variant de PapM selon l'invention présente une efficacité de méthylation 1 de L-PAPA en L-MMPAPA au moins 2 fois supérieure, de préférence au moins 5 fois supérieure et de préférence encore au moins 10 fois supérieure à l'efficacité de méthylation 2 du L-MMPAPA en L-DMPAPA. Un tel variant est plus particulièrement avantageux pour la production de PIB. De préférence, le variant selon l'invention dérive de la séquence du polypeptide PapM sauvage par remplacement d'un ou plusieurs acides aminés choisis parmi : le résidu Gly 249 (a), le résidu Thr 192 (b) ou un résidu équivalent à (a) ou (b) dans un polypeptide homologue.

Par polypeptide homologue de PapM au sens de la présente invention on entend désigner des polypeptides issus ou dérivés de bactéries du genre Streptomyces telles que notamment *Streptomyces olivaceus, Streptomyces ostreogriseus, Streptomyces mitakaensis, Streptomyces loïdensis, Streptomyces graminofaciens, Streptomyces diastaticus* ou d'autres microorganimes dont la séquence d'acide aminés diffère de la séquence d'acide aminé SEQ ID N°1 par substitution, délétion et/ou insertion d'un ou plusieurs acides aminés et dont la fonction biologique est similaire à celle du polypeptide PapM. Une telle séquence de polypeptide homologue est peut être similaire au moins à 60 % de la séquence SEQ ID N°1, de préférence similaire au moins à 70 % et de préference encore similaire au moins à 80 % de la séquence SEQ ID N°1.

Selon une première forme de réalisation, le variant selon l'invention dérive de la séquence polypeptidique SEQ ID N°1 et présente au moins une substitution en position 249 d'une Glycine par une Sérine.

Selon une autre forme de réalisation, le variant selon l'invention dérive de la séquence polypeptidique SEQ ID N°1 et présente au moins une substitution en position 192 d'une Thréonine par une Isoleucine.

Enfin, selon encore une autre forme de réalisation, le variant selon l'invention dérive de la SEQ ID N°1 et présente au moins une substitution en position 249 d'une Glycine par une Sérine et une substitution en position 192 d'une Thréonine par une Isoleucine.

Selon, un autre mode de réalisation, le variant de PapM selon l'invention présente une efficacité de méthylation 2 de L-MMPAPA en L-DMPAPA au moins 2 fois supérieure, de préférence au moins 5 fois supérieure et de préférence encore au moins 10 fois supérieure à l'efficacité de méthylation 1 du L-PAPA en L-MMPAPA. Un tel variant est plus particulièrement avantageux pour accroître la production de PIA.

L'invention concerne également un acide nucléique codant pour un variant du polypeptide tel que défini ci-avant. Il s'agit de préférence, d'un acide nucléique dérivé par mutation de la séquence nucléotidique présentée en SEQ ID N°1 ou les séquences dérivées en raison de la dégénérescence du code génétique. De manière avantageuse, l'acide nucléique selon l'invention, comprend au moins une mutation faux-sens en amont du motif NPPY situé aux positions 193 à 196, et de préférence cette mutation faux-sens entraîne un changement non-conservatif d'acide aminé, tel que par exemple la substitution d'une cytosine en position 658 par une thymine (C658T) (allèle muté 66). Une autre forme de réalisation avantageuse concerne un acide nucléique caractérisé en ce qu'il comprend au moins une substitution d'une guanine en position 828 par une adénine (G828A) (allèle muté 49). Enfin, une autre forme plus particulièrement avantageuse réside dans un acide nucléique caractérisé en ce qu'il comprend au moins une substitution d'une guanine en position 828 par une adénine (G828A) et au moins une substitution d'une cytosine en position 658 par une thymine (C658T) (allèle muté 49/66).

L'invention a également pour objet, l'utilisation d'un acide nucléique tel que défini ci-avant pour modifier la proportion des isoformes des composantes B dans une souche productrice de Streptogramines. A titre de souches productrices de Streptogramines utilisables dans le cadre de l'invention, on peut citer notamment les souches *Streptomyces olivaceus, Streptomyces ostreogriseus, Streptomyces mitakaensis, Streptomyces loïdensis, Streptomyces graminofaciens, Streptomyces diastaticus,* etc. Les acides nucléiques codant pour un variant de PapM qui présente une efficacité de méthylation 1 de PAPA en MMPAPA au moins 2 fois supérieure, de préférence au moins 5 fois supérieure et de préférence encore au moins 10 fois supérieure à l'efficacité de méthylation 2 du MMPAPA en DMPAPA sont plus particulièrement utiles pour la production de PIB lorsqu'ils sont exprimés dans une souche de *S. pristinaespiralis.* Les acides nucléiques codant pour un variant de PapM qui présente une efficacité de méthylation 2 de MMPAPA en DMPAPA au moins 2 fois supérieure, de préférence au moins 5 fois supérieure et de préférence encore au moins 10 fois supérieure à l'efficacité de méthylation 1 du PAPA en MMPAPA sont plus particulièrement utiles pour la production de PIA lorsqu'ils sont exprimés dans une souche de *S. pristinaespiralis.*

L'invention a également pour objet tout ADN recombinant comprenant un acide nucléique tel que défini ci-avant.

L'invention concerne encore tout vecteur d'expression à réplication autonome et/ou intégratif comprenant un acide nucléique ou un ADN recombinant tels que défini ci-avant, tel que notamment un vecteur comprenant tout ou partie du vecteur pVRC1306 représenté sur 1a figure 11.

L'invention a également pour objet les cellules hôtes contenant un acide nucléique et/ou un ADN recombinant et/ou un vecteur d'expression tels que définis ci avant. Les cellules hôtes selon l'invention peuvent être aussi bien des cellules eucaryotes que procaryotes. Parmi les cellules eucaryotes qui conviennent, on peut citer les cellules animales, les levures, ou les champignons. En particulier, s'agissant de levures, on peut citer les levures du genre *Saccharomyces, Kluyveromyces, Pichia, Schwanniomyces,* ou *Hansenula.* S'agissant de cellules animales, on peut citer les cellules COS, CHO, C127, les oeufs de Xénope, etc. Parmi les champignons, on peut citer plus particulièrement *Micromonospora, Aspergillus* ssp. ou *Trichoderma* ssp. Comme cellules procaryotes, on préfère utiliser les bactéries suivantes *Actinomycètes,* et notamment *Streptomyces, E. coli* (exemple 7), *Bacillus.* Préférentiellement, les cellules recombinantes de l'invention sont choisies parmi les cellules productrices de Streptogramines et notamment *Streptomyces olivaceus, Streptomyces ostreogriseus, Streptomyces mitakaensis, Streptomyces loïdensis, Streptomyces graminofaciens, Streptomyces diastaticus.* Les cellules recombinantes de l'invention peuvent être obtenues par toute méthode permettant d'introduire une séquence nucléotidique étrangère dans une cellule. Il peut s'agir notamment de transformation, électroporation, conjugaison, fusion de protoplastes, ou toute autre technique connue de l'homme de l'art.

L'invention a également pour objet un procédé de production d'un variant du polypeptide PapM selon l'invention caractérisé en ce que l'on cultive une cellule hôte telle que définie ci-avant et on récupère le polypeptide produit.

L'invention concerne également l'utilisation d'une cellule hôte telle que définie ci-avant et exprimant un variant du polypeptide PapM selon l'invention dans une réaction de bioconversion. En particulier, ces cellules peuvent permettre de transformer des amines aromatiques en aminés monométhylées, en particulier la 4-amino-L-phénylalanine en 4-méthylamino-L-phénylalanine. Ces bioconversions peuvent se faire soit à l'aide de cellules entières, soit à l'aide d'extraits acellulaires desdites cellules.

Un autre objet de l'invention concerne un procédé de production d'une composante B des Streptogramines caractérisé en ce que :

- on inactive dans une souche productrice de Streptogramines ou potentiellement productrice de Streptogramines le gène papM et on introduit une ou plusieurs copies d'un acide nucléique codant pour un variant de papM tel que défini ci-avant ,
- on cultive ladite souche dans des conditions de production des Streptogramines, et,
- on récupère la composante B des Streptogramines produites.

Préférentiellement, la souche productrice de Streptogramines est une souche productrice de pristinamycines dérivée de *S. pristinaespiralis* et de préférence encore, elle est dérivée de la souche *S. pristinaespiralis* SP92 ou de *S. pristinaespiralis* ATCC25486. Elle peut être aussi choisie parmi les souches suivantes : *Streptomyces olivaceus* ATCC12019, *Streptomices ostreogriseus* ATCC27455, *Streptomyces mitakaensis* ATCC15297, *Streptomyces loïdensis* ATCG11415, *Streptomyces graminofaciens, Streptomyces diastaticus.*

De manière avantageuse, la souche productrice de Streptogramines est une souche qui produit une quantité réduite ou non détectable de composantes A des Streptogramines. A titre de souches ne produisant pas de composantes A des streptogramines on peut citer notamment la souche *S. osteogriseus* Pr4Q031/CBS 142.93 décrite dans la demande WO93/20182. Concernant les exemples de souches de *S. pristinaespiralis* qui ne produisent pas de composantes A, on peut citer la souche SP213 dérivée de SP92 (Blanc et al, 1994) après mutagénèse chimique ou la souche *S.pristinaespiralis* Pr4R31 (déposée CBS182.92) décrite dans la demande WO93/20182 les souches SP213 et Pr4R31 produisent spécifiquement des pristinamycines PI et ne produisent plus de quantité détectable de pristinamycines PII (composantes A).

Selon une variante préférée, l'introduction de l'acide nucléique codant pour un variant de PapM selon l'invention se fait en remplacement de la forme sauvage du gène papM. Selon le variant exprimé par l'acide nucléique introduit, il est possible de favoriser la production de différentes composantes B des streptogramines. Ainsi une souche de *S. pristinaespiralis* exprimant un variant avec un rapport Kcat PAPA. Km MMPAPA / Kcat $_{MMPAPA}$ . Km $_{PAPA}$ augmenté par rapport au polypeptide sauvage conduit à la production préférentielle de PIB. A l'inverse, un variant présentant une diminution de ce rapport doit permettre d'obtenir une production accrue de PIA. Préférentiellement, pour la production de PIB, la souche de *S. pristinaespiralis* comprend un gène papM présentant au moins la mutation (G828A) ou la mutation (C658T) ou la double mutation (G828A) (C658T).

L'invention concerne également un procédé pour la production de PINH2 caractérisé en ce que on utilise un souche dans laquelle la forme sauvage du gène papM de *S. pristinaespiralis* est inactivée et/ou remplacée par un acide nucléique codant une forme inactive du polypeptide PapM.

L'invention a également pour objet une souche mutante de *S. pristinaespiralis* caractérisée en ce qu'elle comprend un acide nucléique codant pour un variant du polypeptide PapM selon l'invention. Une telle souche peut-être obtenue notamment par transformation d'une souche recombinante dont le gène papM sauvage a été disrupté et remplacé par une cassette Ω-Km (cf exemple 8) par un plasmide suicide permettant l'échange de cette cassette par un des allèles mutés de papM. A titre d'illustration, l'invention décrit la souche *S. pristinaespiralis* SP217 comprenant un acide nucléique portant la mutation C658T (allèle mutant 66) ou la souche *S. pristinaespiralis* SP218 comportant la double mutation C658T et G828A (allèle mutant 49/66). Ces mutations ont été introduites dans la souche *S. pristinaespiralis* SP213 préalablement disruptée dans le gène papM et remplacé par une casette Ω-Km. Une approche identique a permis la construction de la souche *S. pristinaespiralis* SP101 avec l'introduction de l'allèle mutant 66 dans la souche SP92. Les souches *S. pristinaespiralis* SP101, SP218 et SP217 qui synthétisent un variant de papM selon l'invention produisent majoritairement de la PIB.

L'invention a également pour objet l'utilisation d'une souche de *S. pristinaespiralis* pour la production de PINH2. Une telle souche est illustrée notamment par la souche SP216 qui dérive de la souche SP213 après inactivation du gène papM et qui produit majoritairement de la PIN2.

L'invention concerne également un procédé de sélection des variants du polypeptide PapM ou d'un polypeptide codé par un gène homologue selon lequel :

- on réalise une étape de de mutagénèse chimique sur le gène papM ou un gène homologue cloné dans un plasmide,
- on réalise une banque en transformant une souche réceptrice avec les plasmides mutagénisés à l'étape (a),
- on sélectionne les clones qui présentent une activité de méthylation tel que le ratio « r » des quantités de substrat de méthylation transformées par unité de temps en conditions de vitesse initiale avec r = Substrat 1 / (Substrat 1 + Substrat 2) est augmenté d'au moins 20 % par rapport au polypeptide sauvage et de préférence supérieur ou égal à 0.6.

A cet égard, la présente invention montre comment il est possible de réaliser *in vitro* une mutagénèse chimique à l'hydroxylamine sur le gène papM cloné dans un plasmide, pour constituer une banque de mutants et pour sélectionner parmi cette banque, à partir d'un criblage enzymatique miniaturisé, les clones de *Escherichia coli* qui expriment les gènes papM mutés dont les protéines correspondantes aient les propriétés catalytiques décrites ci-dessus et qui présentent un rapport « r » tel que défini ci-dessus supérieur ou égal à 0.6.

La présente invention est illustrée à l'aide des exemples qui suivent qui doivent être considérés comme illustratifs et non limitatifs.

LEGENDES DES FIGURES

Figure 1 : exemple de structure de pristinamycines II

Figure 2 : exemple de structure de pristinamycines I

Figure 3 : voie de biosynthèse du MMPAPA et DMPAPA, précurseurs respectifs de la PIB et PIA.

Figure 4 : principe du test enzymatique utilisé pour le criblage des mutants de PapM : marquage par méthylation simultanée du MMPAPA et du DMPAPA à partir respectivement de PAPA et de MMPAPA, en présence de [$^{14}$C-

Me]-SAM.

Figure 5 : représentation du plasmide pVRC706

Figure 6 : représentation du plasmide pUC4K

Figure 7 : séquence et traduction du gène papM sauvage. Positions des mutations identifiées pour les clones mutés 49 et 66.

Figure 8 : représentation du plasmide pVRC721

Figure 9 : stratégie de construction du plasmide pVRC1303

Figure 10: représentation du cas de double crossing over entre le plasmide pVRC1303 et le chromosome de *S. pristinaespiralis.* Structure chromosomique des souches recombinantes.

Figure 11 : stratégie de construction du pVRC1306

Figure 12 : représentation du cas de simple crossing over sélectionné entre le plasmide pVRC1306 et le chromosome de la souche recombinante SP216

Figure 13 : représentation du cas d'un deuxième événement de recombinaison conduisant à une souche recombinante portant un des allèles mutés du gène papM

BIBLIOGRAPHIE

- Blanc et al., (1994) WO94/08014
- Blanc et al., (1995), J. Bacteriol. 177 (18) : 5206-14
- Blanc et al., Mol. Microbiol (1997), 23(2) :191-202
- Chambers et al. (1988) Gene. Aug 15;68(1):139-49.
- Cocito C. G. (1979) Microbiol. Rev., 43 : 145-198.
- Cocito C. G. (1983) In Antibiotics, 6 : (Ed. F. E. Hahn), 296-332.
- Dessen P. C., Fondrat C., Valencien C. et Mugnier C. (1990) Comp. Appl. in Biosciences, 6 : 355-356.
- Di Giambattista M., Chinali G. et Cocito C. G. (1989) J. Antim. Chemother., 24 : 485-507.
- Fellay et al. , (1987) Gene. 52(2-3):147-54.
- Hanahan et al.(1990) PNAS USA 87, pp 4645-4649 (1990)
- Hillemann D., Pülher A. et Wohlleben W. (1991) Nucl. Acids Res., 19 : 727-731.
- Hopwood D. A., Bibb M. J., Chater K. F., Kieser T., Bruton C. J., Kieser H. M., Lydiate D. J., Smith C. P., Ward J. M. et Scrempf H. (1985) A laboratory manual., The John Innes Fondation, Norwich, England.
- Humphreys et al. (1976) Mol Gen Genet. Apr 23;145(1):101-8.
- Maniatis T., Fritsh E. F. et Sambrook J. (1989) Molecular cloning : a laboratory manual. Cold Spring Harbor, N. Y.,
- Messing J., Crea R. et Seeburg P. H. (1981) Nucleic Acids Res., 9 : 309.
- Schluckebier et al. (1995) Gene. May 19;157(1-2):131-4.
- Thibaut et al., (1997) J. Bacteriol. 179(3) :697-704
- Veira et Messing, Gene. 1982 Oct;19(3):259-68.


EXEMPLES

**Exemple 1 : méthodologie et protocole du criblage enzymatique**

Cet exemple illustre le criblage de formes mutées de l'enzyme PapM présentant de nouvelles caractéristiques cinétiques, à partir d'une population d'enzymes issue d'un traitement mutagène à l'hydroxylamine (cf. exemples 2 et 3). En particulier, cet exemple illustre l'obtention de formes mutées de PapM ayant une sélectivité modifiée pour les deux substrats naturels de cette enzyme.

1.1. Méthodologie

La méthylase papM a été précédemment clonée, purifiée et caractérisée (Blanc et al. 1994; Blanc et al. 1997). PapM est une enzyme bifonctionnelle catalysant aussi bien la N-méthylation du L-PAPA en L-MMPAPA que celle du L-MMPAPA en L-DMPAPA (voir figure 4).

Le L-PAPA et 1e L-MMPAPA sont deux substrats de PapM compétitifs, ayant des constantes de Michaelis (Km) respectives de 240 et 530 µM, et pour lesquels la vélocité maximale de PapM (Vmax) est respectivement de 55 et 71 µmoles par heure et par mg de PapM (Blanc et al. 1997).

Le ratio des vitesses V1 et V2 de méthylation respectives du L-PAPA et du L-MMPAPA est dépendant des paramètres cinétiques de PapM (Km1 et Vmax1 pour L-PAPA, Km2 et Vmax2 pour L-MMPAPA), et également dépendant des concentrations S1 et S2 respectives du L-PAPA et du L-MMPAPA selon la formule:

$$V1/V2=( Vmax1/Km1 )[S1]/( Vmax2/Km2 )[S2]$$

La mesure simultanée des deux vitesses de méthylation V1 et V2 à des concentrations fixes de S1 et S2 permet de déterminer le ratio des constantes cinétiques de PapM pour le L-PAPA et le L-MMPAPA. Ce ratio est indépendant de la quantité de PapM mise en jeu dans l'essai. Lorsque la sélectivité de l'enzyme PapM mutée augmente pour le L-PAPA, le ratio V1/V2 doit augmenter, et inversement ce ratio doit diminuer lorsque l'enzyme PapM mutée méthyle préferentiellement le L-MMPAPA.

Enfin, pour s'affranchir de faibles variations de Km et privilégier au contraire soit des fortes variations de Km, soit des modifications des Vmax, les concentrations en L-PAPA et L-MMPAPA ont été fixées à environ 6 fois le Km afin d'être en conditions de saturation. Dans de telles conditions, avec l'enzyme PapM sauvage, les vitesses de méthylation 1 et de méthylation 2 sont pratiquement égales.

Le dosage simultané des deux activités de méthylation de PapM est décrit dans l'exemple 7.1. Ce dosage des activités méthylase de PapM est effectué en présence de 1,5 mM L-PAPA, 3 mM L-MMPAPA et 70 µM SAM.

1.2. Criblage de la population d'enzymes PapM mutées

Les plaques 96 puits de la banque de clones de *E. coli* produisant les enzymes PapM mutées, préparées comme décrit à l'exemple 3, sont décongelées à 27°C. Ensuite, aux 200 µl de la culture sur milieu LB contenue dans chaque puits sont ajoutés 25 µl du mélange A. Le mélange A est obtenu en ajoutant successivement (1) 3 ml de tampon 50 mM Bis-Tris-Propane pH 10 dans lequel ont été dissouts 12 mg de L-PAPA, et 71 mg de (DL)-MMPAPA sous forme de dichlorhydrate et préparé comme décrit dans WO 9601901-A1 , (2) 0,7 ml de SAM 4 mM dans de l'eau , (3) 0,7 ml de [$^{14}$C-Me]-SAM à 25 µCi/ml et 60 mCi/mmole (AMERSHAM) (4) 0,6 ml de tampon 100 mM Bis-Tris-Propane pH 6,8, (5) 56 mg d'EDTA, sel di-sodique. Les quantités sont données pour la préparation d'un volume de 5 ml de mélange A. La plaque est ensuite scellée avec un film adhésif, puis incubée à 27°C pendant 2 heures. La réaction est arrêtée avec 40 µl d'une solution d'arrêt. La solution d'arrêt (pour un volume de 4 ml) est obtenue en mélangeant 2,2 ml d'une solution à 24 mg/ml d'heptane sulfonate de sodium dans l'eau avec 1,8 ml d'acide chlorhydrique concentré. Après centrifugation des plaques à 3000 g pendant 5 min, environ 100 µl du surnageant de chaque puits sont analysés par CLHP dans le système décrit à l'exemple 7.1.

Pour chaque puits, les aires respectives A1 et A2 des pics de MMPAPA et de DMPAPA issus de l'enregistrement de la détection radiochimique sont intégrées. Un ratio r est alors calculé de la façon suivante :

$$r = A1 / (A1+A2)$$

Dans le cas de la méthylase PapM sauvage, r = 0,50 $\pm$ 0,05.

Le seuil du ratio r fixé pour une enzyme PapM mutée significativement plus active pour la méthylation 1 que la méthylation 2 est d'environ 0,60.

**EXEMPLE 2 : Mutagénèse à l'hydroxylamine du gène papM**

Cet exemple illustre la mutagénèse du gène papM à partir d'un ADN plasmidique de *Streptomyces* cloné dans un plasmide pouvant se répliquer chez *E. coli.*

2.1. Choix de l'agent mutagène

Une mutagénèse chimique a été réalisée en utilisant l'hydroxylamine (NH2OH) qui se lie spécifiquement aux résidus cytosines pour former des N4-hydroxycytosines qui sont alors capables de s'apparier avec les résidus adenines. L'hydroxylamine provoque donc des transitions G:C > A:T. Le choix de cet agent mutagène est particulièrement intéressant pour effectuer des mutations des ADN riches en GC tel que l'ADN de *Streptomyces* qui contient 70 à 75% de résidus GC.

2.2 Choix des plasmides à mutagéniser

La mutagénèse a été réalisée simultanément sur deux plasmides différents, le plasmide pVRC706 et le plasmide pUC4K.

Le plasmide pVRC706 (Figure 5) est un dérivé de pMTL23 (Chambers et al., 1988) dans lequel a été cloné entre les sites uniques MauI et BamHI (l'extrémité coupée par BamHI ayant été rendue franche par l'action de l'enzyme Kleenow selon le protocole de Sambrook et al. (1989)), le fragment MluI-StuI de 1,7 kb issu du plasmide pVRC409 portant le gène papM à mutagéniser (Blanc et al., 1994; Blanc et al, 1997). Le clonage de l'extrémité StuI dans le site BamHI de pMTL23 rempli à l'aide de l'enzyme Kleenow permet de reconstituer le site BamHI qui peut être réutilisé lors de clonages ultérieurs. Le clonage au site MluI permet d'obtenir une fusion en phase entre les 32 premiers acides aminés de la β-galactosidase codée par le gène lacZ du plasmide pMTL23 et les 11 derniers acides aminés du gène papB situé en amont du gène papM, ceci préserve le couplage traductionnel qui semble exister entre ces deux gènes au vu de la séquence nucléotidique (Blanc et al., 1994). Dans cette construction l'expression du gène papM est contrôlée par le promoteur Plac du gène lacZ inductible par l'ajout de 1mM d'IPTG. Il a préalablement été montré qu'un tel système d'expression permet de produire dans *E. coli,* la méthylase PapM sous la forme d'une protéine active et soluble représentant environ 0,5% des protéines totales (Blanc et al., 1994).

Le plasmide pUC4K (Figure 6) est un dérivé du vecteur pUC4 portant les deux gènes conférant la résistance à l'ampicilline et à la kanamycine (Vieira et Messing, 1982). Ce plasmide a été choisi comme témoin de mutagénèse pour les raisons suivantes: (1) sa taille (3,9 kb) est proche du plasmide pVRC706 (4,4 kb), (2) les deux plasmides pUC4K et pVRC706 ont une structure et des propriétés très proches puisqu'ils dérivent tous les deux des vecteurs de la série pUC et (3) la présence de deux gènes de résistance ampR et kanR portés par pUC4K permet de calculer aisément le pourcentage de clones ayant subi une mutagénèse dans l'un de ces gènes en comptabilisant les clones sensibles à l'antibiotique choisi.

2.3. Protocole de mutagénèse

Deux mutagénèses à l'hydroxylamine ont été réalisées selon le protocole de Humprey et al (1976) en faisant varier la température d'incubation : 80°C et 85°C : 5 mg d'ADN plasmidique purifié sur gradient de chlorure de césium sont mis au contact de 0,4 M d'hydroxylamine (pH 6) dans un volume final de 100 ml pendant 35 minutes à la température désirée (80°C ou 85°C) dans un tampon contenant 0,05 M de phosphate de sodium et 0,5 mM d'EDTA. La réaction de mutagénèse est stoppée par l'ajout de 100 ml de tampon contenant 0,1 M de phosphate de sodium et 1 mM d'EDTA et l'hydroxylamine est éliminée par huit bains de dialyse successifs contre 2 litres de tampon TES (10 mM Tris pH 7,5, 1 mM EDTA et 100 mM NaCl). L'ADN ainsi traité est précipité par l'ajout de 1/10 de volume d'acétate de sodium 3M (pH 8) et 2,5 volumes d'éthanol à 95%. Le culot d'ADN est rincé, séché puis repris dans 100 μl de tampon TE (10 mM Tris-HCl et 1 mM EDTA, pH 7,5). Pour chaque plasmide, il est effectué un controle de la réaction de mutagénèse à la température étudiée en absence d'hydroxylamine.

2.4. Préparation de la souche *E. coli* préceptrice des plasmides mutagénisés

La souche *E. coli* DH5α (supE44 DlacU169 (f80lacZDM15) hsdR17 recA1 endA1 gyrA96 thi-1 relA1), (Hanahan, 1990) a été utilisée pour évaluer la mutagénèse après transformation par les plasmides pVRC706 et pUCK4 mutagénisés selon le protocole décrit ci-dessus.

Les cellules de *E. coli* DH5α compétentes pour la transformation par électroporation ont été préparées selon le protocole suivant : une culture de 1 litre en milieu LB est réalisée à 37°C jusqu'à ce que la D. O (à 600 nm) atteigne 0,5 à 0,8. Les cellules sont ensuite placées sur glace pendant 30 minutes pour stopper leur croissance puis centrifugées à 4°C à 4000 g pendant 15 mn. Le culot de cellules est successivement rincé avec 1 litre d'eau stérile froide, 0,5 litre d'eau froide et 20 ml d'une solution froide contenant 10 % de glycérol. Après chaque rinçage, les cellules sont centrifugées à 4°C à 4000 g pendant 15 mn. En final, le culot cellulaire est repris dans 2 ml de glycérol 10 %. Les cellules ainsi préparées peuvent être immédiatement électroporées ou bien congelées et stockées à -80°C sous forme d'aliquots de 40 ml en vue d'une utilisation ultérieure. L'efficacité de transformation de cellules electrocompétentes est de l'ordre de

$10^9$ colonies/µg d'ADN plasmidique de pUC18.

Les cellules *E. coli* DH5α ainsi préparées ont été électroporées avec l'équivalent de 5 ng de plasmide à tester. Pour ce faire, 40 µl de cellules électrocompétentes sont mis en contact pendant 1 min à 4˚C avec 1 à 2 µl d'une solution contenant 5 ng d'ADN plasmidique. L'électroporateur (Bio-Rad) est réglé pour un pulse de 25 mF et 2500 V. La résistance du controleur de pulse est fixée à 200 Ω. Le mélange cellules/ADN est placé dans une cuvette d'électroporation d'une largeur de 0,2 cm et le pulse électrique est appliqué. On obtient classiquement un temps de pulse de 4,5 à 5 ms. Après le pulse, on ajoute rapidement aux cellules 1 ml de milieu SOC (2% bacto-agar, 10 mM NaCl, 2.5 mM KCl, 10 mM MgCl2, 10 mM Mg SO4, 20 mM glucose) et on transfère le tout dans un tube de 5 ml en polypropylène qui est incubé 1 heure à 37˚C. Cette étape permet aux cellules de récupérer et de commencer à exprimer le ou les gènes de résistance présents sur les plasmides qui ont pénétré les cellules. A la fin de l'incubation, la culture de cellules est diluée de manière appropriée (les facteurs de dilution sont classiquement de $10^{-1}$ à $10^{-4)}$ et les colonies transformées sont sélectionnés sur milieu LB solide contenant l'antibiotique de sélection (50 µg/ml d'ampicilline ou 50 µg/ml de kanamycine).

2.5 Evaluation de la mutagénèse et détermination de la condition de température retenue

Les deux conditions de température de mutagénèse testées ont été évaluées selon les deux critères suivants : d'une part la capacité des plasmides mutagénisés à transformer une souche de *E. coli* et d'autre part, le pourcentage de clones de *E. coli* transformés avec les plasmides pUC4K mutagénisés ayant perdu l'une des deux résistances codées par les plasmides.

Pour les deux plasmides pVRC706 et pUCK4, il a été mesuré la capacité des plasmides mutagénisés à l'hydroxylamine à transformer la souche *E. coli* DH5α. Cette capacité a été comparée à celle des plasmides non mutagénisés réalisés comme contrôle de la température d'incubation en absence d'hydroxylamine. En effet l'action de l'hydroxylamine doit engendrer des mutations léthales dans les plasmides, et plus particulièrement au niveau des gènes de résistance permettant la sélection des clones recombinants ainsi que dans les régions portant l'origine de réplication. On peut donc considérer que la perte de la capacité à transformer d'un plasmide est d'autant plus importante que le traitement de mutagénèse a été efficace. Les capacités à transformer des deux plasmides mutagénisés exprimées en % de la capacité des plasmides non mutagénisés témoins sont indiquées dans le tableau ci-dessous.

Tableau 1

| | pVRC 706 | | pUC4K | |
|---|---|---|---|---|
| | 80 ˚C | 85 ˚C | 80˚C | 854˚C |
| Ampicilline | 12,5 | 1,5 | 14 | 1,5 |
| Kanamycine | - | - | 16 | 0,3 |

Ces résultats indiquent clairement que la mutagénèse à 85˚C est nettement plus efficace pour les deux plasmides pVRC706 et pUC4K puisqu'elle altère statistiquement dix fois plus d'entités plasmidiques, de telle façon que seulement 1,5 % des plasmides sont encore capables de transformer des cellules électrocompétentes de *E. coli* DH5α, de se répliquer et de conférer aux clones résultants la résistance à l'ampicilline. De plus, les résultats observés avec le pUC4K sont très homogènes quelque soit la sélection choisie ($Ampi^R$) ou ($Kana^R$) ce qui indique que l'action de l'hydroxylamine s'est produit de manière homogène dans les régions essentielles à l'expression de ces deux résistances.

Le deuxième critère d'évaluation a consisté à calculer, pour les plasmides mutagénisés pUC4K, le % de clones sensibles à la kanamycine ($Kana^S$) parmi les clones résistants à l'ampicilline ($Ampi^R$) et réciproquement. Ces pourcentages reflètent la fréquence des mutations qui se sont produites, sous l'effet de l'hydroxylamine, au niveau des gènes de résistance considérés (ampR ou kanR) et qui ont perturbé leur expression soit par blocage complet (arrêt de la transcription ou de la traduction), soit en conduisant à la synthèse d'une protéine tronquée et/ou inactive.

Pour estimer ces pourcentages, 200 clones ($Kana^R$) ou ($Ampi^R$) choisis parmi les clones obtenus après transformation de cellules électrocompétentes de la souche de *E. coli* DH5α par les plasmides pUC4K mutagénisés ou non, ont été repiqués sur milieu LB solide contenant respectivement de l'ampicilline ou de la kanamycine. Les clones sensibles à l'un des deux antibiotiques ont été comptabilisés après 24 h de croissance. Les pourcentages de clones sensibles à l'ampicilline ou à la kanamycine parmi les clones transformés par les plasmides pUC4K mutagénisés, ou non, à l'hydroxylamine sont reportés dans le tableau ci-dessous.

Tableau 2

| | pUC4K + hydroxylamine | | pUC4K non mutagénisés | |
|---|---|---|---|---|
| | 80°C | 85°C | 80°C | 85°C |
| $(Ampi^S)/(Kana^R)$ | 1,3 | 3,2 | 0 | 0 |
| $(Kana^S)/(Ampi^R)$ | 1,5 | 6 | 0 | 0 |

Ces résultats permettent de conclure que seul le traitement à l'hydroxylamine a bien engendré des mutations affectant l'expression d'un des deux gènes de résistance du pUC4K. Ils permettent de conclure également que la mutagénèse réalisée à 85°C a été 3 à 4 fois plus efficace pour les deux gènes de résistance des plasmide pUC4K que la mutagénèse effectuée à 80°C. Enfin ces résultats montrent que l'action de l'hydroxylamine a été relativement homogène pour les deux gènes de résistance ampR et KanR.

On peut donc raisonnablement supposer que l'action de l'hydroxylamine s'est produite sur l'ensemble des deux plasmides pUC4K et pVRC706 et que la mutagénèse effectuée à 85°C est probablement celle qui a le plus touché les gènes papM portés par les pVRC706. Cette comparaison des conditions de mutagénèse nous a conduit à ne retenir pour la suite de l'étude que les clones résultant de la transformation des cellules électrocompétentes de la souche *E. coli* DH5α par les plasmides pVRC706 issus de la mutagénèse à l'hydroxylamine réalisée à 85°C.

**Exemple 3 : constitution et exploitation d'une banque de *E. coli* DH5α hébergeant des plasmides pVRC706.**

Cet exemple illustre la construction et l'exploitation d'une banque de 6000 clones de la souche *E. coli* DH5α hébergeant des plasmides ayant subi une mutagénèse chimique à l'hydroxylamine réalisée à 85°C.

Dans le cas de la mutagénèse à l'hydroxylamine réalisée à 85°C sur les plasmides pVRC706 portant le gène papM comme il est décrit plus haut, on peut évaluer, en faisant l'hypothèse que l'action de l'hydroxylamine a été générale sur l'ensemble des plasmides pVRC706 (cf Exemple 2), qu'une banque de 6000 clones de *E. coli* hébergeant ces plasmides représente une banque de mutants dont un certain pourcentage des clones expriment un gène papM muté. L'analyse des 6000 clones de la banque par dosage simultané des deux activités de méthylations catalysées par les protéines PapM correspondantes confirme cette hypothèse (voir exemple 4).

Pour constituer cette banque, 6000 clones sélectionnés sur des boites de milieu LB contenant 50 mg/ml d'ampicilline après transformation de cellules électrocompétentes de la souche *E. coli* DH5α sont repiqués individuellement dans des puits de plaques de microtitration à 96 cellules contenant 200 ml du milieu de culture et conservation Hogness (Sambrook et al. 1989) additionné de 100 μg/ml d'ampicilline. Les plaques mères ainsi constituées sont mises à incuber à 37°C sous une agitation modérée de 160 rpm pendant 24h de manière à ce que les clones se multiplient et forment des suspensions saturées de bactéries ayant atteint la phase stationnaire de croissance. On procède alors à la réplique en double exemplaire des plaques mères en réensemençant individuellement chaque puit de deux plaques de 96 cellules contenant 200 ml de milieu de culture LB additionné de 100 μg/ml d'ampicilline et 1 mM d'IPTG à partir d'un aliquot des cultures de chaque puit des plaques mères. Ces plaques ainsi obtenues, appelées plaques de dosage, sont mises à incuber pendant 24h (37°C, 160 rpm) de manière à ce que les clones se multiplient et forment des suspensions saturées de bactéries ayant atteint la phase stationnaire de croissance. La présence des plasmides pVRC706 dans les cellules bactériennes est sélectionnée par l'ampicilline et l'expression des gènes de méthylase papM portés par ces plasmides est induite par l'ajout d'IPTG en début de culture. Ces plaques sont ensuite congelées à -80°C avant le dosage des activités de méthylation qui est effectué sur les suspensions bactériennes brutes (voir exemple 1).

Les plaques mères, une fois répliquées en double exemplaire, sont conservées à -80°C et constituent une forme de stockage à long terme des 6000 clones à analyser.

**Exemple 4 : résultats du screening de la banque de *E. coli* DH5α hébergeant des plasmides pVRC706 mutagénisés.**

Cet exemple illustre l'analyse rapide d'une banque de 6000 clones à partir d'un test d'activité miniaturisé afin de sélectionner les clones hébergeant des gènes mutés codant pour des enzymes ayant des propriétés intéressantes.

4.1. Résultats bruts du criblage des 6000 clones

A l'issue du premier criblage, effectué comme décrit à l'exemple 1, sur les 6000 clones de la banque préparée à l'exemple 3, on sélectionne:

- 441 clones présentant une activité de méthylation nulle ou indétectable, soit un taux de 7,3 %.

- 9 clones présentant une première réaction de méthylation favorisée par rapport à la deuxième réaction, c'est à dire des clones ayant un rapport r (comme défini à l'exemple 1.2) supérieur à 0,6. La fréquence d'apparition de tels mutants est donc très faible, elle est de 0,15 %.

Ces premiers résultats sont vérifiés comme décrit ci-dessous.

4.2. Méthodologie de confirmation des clones sélectionnés

Les activités de méthylation de tous les clones sélectionnés au cours du premier criblage sont systématiquement redosées à partir des plaques de réplique préparées comme à l'exemple 3, et en suivant le même protocole de manière à confirmer les résultats des premiers dosages. Il apparaît que pour la grande majorité des clones, les résultats de dosage sont confirmés.

Ainsi on confirme que:

- 360 clones présentent une activité de méthylation nulle, soit un taux de 6 %.

- 8 clones présentent une première réaction de méthylation favorisée par rapport à la deuxième réaction, c'est à dire des clones ayant un rapport r (comme défini à l'exemple 1.2) supérieur à 0,6. La fréquence d'apparition de tels mutants est donc très faible, elle est de 0,13 %.

Pour les 8 clones dont le rapport des efficacités enzymatiques est tel que r > 0.60, deux autres étapes de confirmation sont mises en place :

(1) L'ADN plasmidique de ces clones est isolé à partir de techniques standard (Sambrook et al., 1989) et réintroduit dans des cellules fraiches électrocompétentes de la souche *E. coli* DH5$\alpha$. Cette étape permet de vérifier que l'altération du ratio r des activités de méthylation observé chez les 8 clones n'est pas liée au contexte chromosomique des clones mais dépend uniquemment des plasmides provenant des clones sélectionnés.

(2) L'étape suivante de vérification consiste à recloner dans des vecteurs d'expression frais pMTL23 les gènes papM présents dans les clones sélectionnés. Pour cela, on isole les fragments MluI-BamHI de 1.7kb portant les gènes papM à partir de l'ADN plasmidique extrait des clones sélectionnés que l'on reclone entre les sites uniques MluI et BamHI de pMTL23. Les étapes nécessaires à ces clonages (isolement d'ADN plasmidique, digestion d'ADN plasmidique par enzyme de restriction, migration et analyse de digestions enzymatiques par électrophorèse en gels d'agarose, préparation de fragments d'ADN après migration électrophorétique, réactions de ligation) ont été faites selon les protocoles standards décrits par Sambrook et al. (1989).

Les plasmides résultant ont une structure analogue à celle de pVRC706 (Figure 5) à ceci près qu'ils portent un des 8 allèles mutés du gène papM. Ils sont appelés pVRC710 à pVRC717, chaque numéro étant attribué à l'un des 8 allèles mutés et sont réintroduits dans des cellules fraiches électrocompétentes de la souche *E. coli* DH5$\alpha$. Les activités de méthylation des clones résultants sont redosées comme il est décrit ci-dessus. Cette étape permet de vérifier que l'altération du ratio r des activités de méthylation observée dans ces clones n'est pas lié à une (ou plusieurs) mutation (s) qui se serai(en)t produite(s) dans une région de pVRC706 extérieure au gène papM mais dépend uniquement d'une (ou de plusieurs) mutation(s) localisée(s) dans le gène papM.

Toutes les étapes de vérification permettent de confirmer le phénotype de 7 des 8 mutants préalablement sélectionnés. Par exemple, on observe que le ratio des deux activités de méthylation calculé comme il est décrit à l'exemple 1.2. est de 0.63 pour le mutant 49 (clone 49C9) et 0.67 pour le mutant 66 (clone 66A9) alors qu'il n'est que de 0.40 pour le clone exprimant le gène sauvage papM dans la même expérience.

**Exemple 5 : Caractérisation génétique des gènes mutants papM issus de la mutagénèse réalisée à l' hydroxylamine à 85°C sur les plasmides pVRC706**

Cet exemple illustre la caratérisation génétique des gènes ayant subi la mutagénèse chimique. La caractérisation génétique est faite par clonage et séquençage des gènes mutés afin de comparer leur séquence à celle du gène sauvage et mettre en évidence la (ou les) mutations rendant compte des phénotypes observés. Cette caractérisation génétique est menée parallèlement à une caractérisation biochimique des protéines codées par les gènes mutés (cf exemple 7) afin de corréler le (ou les) changements d'acides aminés observés aux modifications des propriétés catalytiques constatées pour ces enzymes.

5.1. Méthode

Dans le cas des gènes papM issus de la mutagénèse réalisée à l'hydroxylamine à 85°C sur les plasmides pVRC706, on a réalisé le sous-clonage et le séquençage des gènes papM des 7 clones sélectionnés. La partie de la région MluI-BamHI de 1,7 kb des plasmides pVRC706 mutagénisés portant les gènes mutés papM a été sous-clonée en deux sous-inserts dans des vecteurs dérivés du phage M13 (Messing et al., 1981) adaptés au séquençage d'ADN par la méthode de Sanger (Sambrook et al., 1989) : le fragment SmaI-AgeI de 0,7 kb contenant la partie 3' du gène papM a été cloné au site SmaI du vecteur M13mp18 et le fragment SalI-PstI de 0,4 kb contenant la partie 5' a été cloné entre les sites SalI et PstI des vecteurs M13mp18 et M13mp19 (Figure 5). Les étapes nécessaires à ces sous-clonages ont été faites selon les protocoles standards décrits par Sambrook et al. (1989). Le séquençage de ces deux inserts a été réalisé sur double brin par la méthode de terminaison de chaîne dite de Sanger utilisant des dideoxynucléotides fluorescents du système PRISM ready Reaction DyeDeoxy Terminator Cycle Sequencing Kit commercialisé par Applied Biosystems. Les réactions de séquençage sont effectuées dans un appareil de P.C.R. sur de l'ADN simple brin en utilisant comme amorce de synthèse le primer universel (Sambrook et al., 1989) ou des oligonucléotides complémentaires d'une séquence de 20 nucléotides de l'insert à séquencer et synthétisés par un synthétiseur automatisé Biosearch 8600 (Blanc et al, 1995). Les produits de ces réactions sont ensuite analysés par un séquenceur automatique Applied Biosystems 370A qui assure la migration électrophorétique et la lecture des séquences. Les séquences sont ensuite traitées grâce aux programmes du serveur BISANCE du centre de Bioinformatique Infobiogen (Dessen et al., 1990) qui permettent entre autre leur concaténation, leur traduction en protéine, la comparaison avec la séquence sauvage.

5.2. Résultats

Dans un premier temps les 7 clones suivants ont été séquencés sur simple brin et le ratio r a été redéterminé (dans une expérience pour laquelle le ratio de l'enzyme PapM sauvage a été trouvé égal à 0,40)

| mutant | ratio | Mutation | Mutation (Nucl.) |
|---|---|---|---|
| 45 | 0,52 | Thr274>Ile | ACC>ATC |
| 49 | 0,63 | Gly249>Ser | GGC>AGC |
| 52 | 0,66 | Gly249>Asp | GGC>GAC |
| 66 | 0,67 | Thr192>Ile | ACC>ATC |
| 69 | 0,51 | Met226>Ile | ATG>ATA |
| 74D4 | 0,63 | Gly249>Ser | GGC>AGC |
| 74D5 | 0,52 | Met226>Ile | ATG>ATA |

Les résultats de la comparaison des séquences des protéines codées par les gènes mutés papM issus de la mutagénèse réalisée à l'hydroxylamine à 85°C sur les plasmides pVRC706 avec la séquence de la protéine codée par le gène sauvage papM (Blanc et al., 1994; Blanc et al, 1997) sont indiqués sur la figure 7 et dans le tableau suivant, pour 2 des 7 clones sélectionnés dont la séquence a été vérifiée en double brin.

Tableau 3

| | mutant 49 (clone 49C9) | mutant 66 (clone 66A9) |
|---|---|---|
| mutation nucléotidique dans le gène papM | GGC > AGC | ACC > ATC |
| substitution d'acide aminé dans la protéine PapM | $Gly_{249}$ > Ser | $Thr_{192}$ > Ile |

Dans ce tableau, seules sont indiquées les mutations qui provoquent un changement d'acides aminés de la protéine correspondante. Pour certains clones, et en particulier pour le clone 66A9 nommé mutant 66, on a observé d'autres mutations dites silencieuses qui ne conduisent pas à de changement de la séquence en acides aminés du fait de la dégénérescence du code génétique.

On constate que pour chacun des 7 clones sélectionnés et séquencés, on peut mettre en évidence une mutation ponctuelle dans le gène papM conduisant au changement d'un acide aminé et correspondant effectivement aux types de changement C > T ou G > A attendus après un traitement à l'hydroxylamine. Parmi les 7 clones analysés, on retrouve plusieurs fois certaines de ces mutations de telle sorte qu'on peut regrouper ces 7 clones en 4 classes de mutants selon la position touchée. Les clones décrits ci-dessus représentent 2 des 4 classes ainsi constituées.

Les effets de deux mutations ont été plus particulièrement étudiées dans la suite de ce travail, il s'agit, par référence à la séquence SEQ ID N°1, d'une part de la substitution d'une cytosine en position 658 par une thymine (C658T) (allèle muté 66) et d'autre part de la substitution d'une guanine en position 828 par une adénine (G828A) (allèle muté 49).

Parallèlement à cette caractérisation génétique, la caractérisation biochimique des protéines codées par les gènes mutés telle que décrite dans l'exemple 7, a permis d'observer un changement des propriétés catalytiques de ces enzymes par rapport à la protéine codée par le gène sauvage papM.

Il est ainsi possible de corréler les changements d'acides aminés observés aux modifications des propriétés catalytiques constatées pour ces enzymes.

On remarque ainsi que pour le mutant 66, l'acide aminé modifié est la thréonine située en position 192 juste avant le motif NPPY situé aux positions 193 à 196 (Figure 7). Ce motif est conservé chez toutes les N-méthylases et plus particulièrement les N-méthylases d'ADN (méthylant les positions 6 des adenines et 4 des cytosines) et ferait partie du domaine catalytique de ces enzymes (Schluckebier et al., 1995). La modification du résidu thréonine, un acide aminé polaire, en résidu isoleucine qui est hydrophobe, est donc vraisemblablement importante pour les propriétés catalytiques de la protéine correspondante. On a en effet constaté un changement important des propriétés catalytiques de l'enzyme codée par le gène muté 66 et plus particulièrement pour la deuxième réaction de méthylation (cf exemple 7).

**Exemple 6 : construction par génie génétique de gènes papM doublement mutés à partir des gènes mutés papM issus de la mutagénèse réalisée sur les plasmides pVRC706**

Cet exemple illustre la construction par génie génétique de gènes doublement mutés à partir de gènes présentant des mutations simples obtenus après une mutagénèse réalisée à l'hydroxylamine sur ADN plasmidique (cf exemple 2), sélectionnés par criblage enzymatique (cf exemple 4) et caractérisés par séquençage (cf exemple 5). De manière surprenante, cette stratégie a permis d'obtenir des gènes mutés plus intéressants cumulant les propriétés observées chez les simples mutants.

Plusieurs combinaisons de mutations prises deux à deux ont été effectuées. A titre d'exemple, la combinaison des mutations 49 et 66 est détaillée ci-dessous. A cet effet, un gène chimère a été réalisé en accolant la partie 5' du gène muté papM 66 avec la partie 3' du gène muté papM 49. Pour ce faire, on isole les fragments d'ADN suivants :

- le fragment MluI-XhoI de 0,65 kb est isolé à partir du plasmide pVRC713 3 portant le gène muté papM 66 (cf exemple 4). Ce fragment contient le début de l'allèle 66 y compris le codon ATC qui a été muté et qui code pour l'isoleucine que l'on trouve en position 192 de la méthylase modifiée 66 (voir figure 7).

- le fragment XhoI-BamHI de 1,05 kb est isolé à partir du plasmide pVRC711 portant le gène muté papM 49 (voir exemple 4). Ce fragment contient la fin de l'allèle 49 y compris le codon AGC qui a été muté et qui code pour la serine que l'on trouve en position 249 de la méthylase modifiée 49 (voir figure 7).

Ces deux fragments sont co-clonés selon les protocoles standards de Sambrook et al. (1989) entre les sites uniques MluI-BamHI du plasmide pMTL23 (Chambers et al., 1988) de manière à reconstituer un plasmide ayant une structure analogue à celle de pVRC706 (figure 5), à ceci près qu'il porte l'allèle doublement muté du gène papM codant pour une protéine modifiée aux positions 192 et 249. Le plasmide résultant est nommé pVRC718 et permet l'expression du gène doublement muté 49/66 après transformation de cellules électrocompétentes de la souche *E. coli* DH5$\alpha$ et induction du promoteur Plac par ajout de 1mM d'IPTG.

L'allèle doublement muté 49/66 est ensuite transféré dans un deuxième vecteur d'expression pET11c (Novagen) utilisant le promoteur fort PT7 reconnu par l'ARN polymérase du phage T7. Pour ce faire, on isole à partir du plasmide pVRC718 le fragment BglII-BamHI de 1,8 kb portant le gène papM 49/66 que l'on clone au site unique BamHI de pET11c. On sélectionne un plasmide recombinant nommé pVRC 721 décrit sur la figure 8 correspondant au sens d'insertion de manière à réaliser une fusion traductionnelle entre le gène 10 du phage T7 et le gène papB situé en amont du gène papM. Ce plasmide est ensuite introduit dans la souche d'expression *E. coli* B121(DE3) permettant l'expression de l'ARN polymérase du phage T7 et donc du gène papM 49/66 transcrit à partir du promoteur T7.

Avec une construction similaire utilisant le système d'expression du phage T7, il a été observé une surproduction de la protéine PapM sauvage 10 fois plus importante que la production obtenue avec le plasmide pVRC706 (Blanc et al., 1997). Le système utilisant le phage T7 permet de même de surproduire la méthylase modifiée codée par le gène doublement muté 49/66 en vue de sa purification et de sa caractérisation comme il est décrit dans l'exemple 7.

**Exemple 7 : caractérisation biochimique du produit du gène papM (sauvage ou muté) de *S. pristinaespiralis* exprimé dans des souches de *E. coli.***

Cet exemple illustre la détermination des constantes cinétiques des réactions catalysées par la protéine PapM (mutée ou sauvage). La protéine PapM catalyse deux réactions, d'une part la méthylation de la 4-amino-L-phénylalanine (L-PAPA) en 4-méthylamino-L-phénylalanine (L-MMPAPA) et d'autre part, la méthylation de la 4-méthylamino-L-phénylalanine en 4-diméthylamino-L-phénylalanine (L-DMPAPA).

7.1. Dosage de l'activité de méthylation de la 4-amino-L-phénylalanine en 4-méthylamino-L-phénylalanine et de l'activité de méthylation de la 4-méthylamino-L-phenylalanine en 4-diméthylamino-L-phénylalanine.

Cet exemple illustre le dosage de deux activités terminales de la biosynthèse de la 4-diméthylamino-L-phénylalanine, composant de la pristinamycine IA. Il s'agit de la méthylation de la 4-amino-L-phénylalanine en 4-méthylamino-L-phénylalanine (méthylation 1) d'une part, et de la méthylation de la 4-méthylamino-L-phénylalanine en 4-diméthylamino-L-phénylalanine (méthylation 2) d'autre part ; ces deux activités utilisant la SAM comme donneur de groupement méthyle (figure 4).

Les fractions enzymatiques à doser (1 à 20 unités) sont incubées pendant 30 min à 27°C dans un volume total de 200 µl de tampon pH 6.8 bis-tris-propane 50mM contenant de la SAM (200 µM) dont le groupement méthyle est marqué radioactivement avec l'isotope 14 de l'atome de carbone (2 Ci/mol), en présence de 4-amino-L-phénylalanine (1 mM) pour le dosage de la méthylation 1 ou de 4-méthylamino-L-alanine (2.5 mM) pour le dosage de la méthylation 2.

La réaction est arrêtée par ajout de 16 µl d'acide chlorhydrique à 37% puis de 20 µl d'heptanesulfonate de sodium à 240 g/l. Après centrifugation, 150 µl de surnageant sont injectés dans le système de CLHP en mode gradient suivant :

- Phase mobile : Eluant A=1,2 g d'heptanesulfonate+2,5 ml d'acide acétique glacial + eau (qsp 1000 ml). Eluant B = 1,2 g d'heptanesulfonate de sodium + 2,5 ml d'acide acétique glacial + 300 ml d'acétonitrile + eau (qsp 1000 ml). Le gradient est réalisé de la façon suivante à t = 0 , 30 % d'éluant B ; t = 16 (min.), 30 % d'éluant B ; t = 17 (min.), 100 % d'éluant B ; t = 20 min. , 100 % d'éluant B ; t = 21 min., 30 % d'éluant B ; t = 25 (min.), 30 % d'éluant B.

- phase stationnaire : colonne Nucléosil® C18 5 µm 150 x 4,6 mm (Macherey-Nagel)

En sortie de colonne, les substrats et produits de la réaction enzymatique sont quantifiés par absorption à 254 nm. Cette détection est couplée à une détection radiochimique en ligne au moyen d'un détecteur Berthold LB506 équipé d'une cellule à scintillation solide de type GT400-U4. Ceci permet de suivre spécifiquement l'incorporation de groupements méthyles radioactifs dans les produits de la réaction.

L'unité d'activité enzymatique pour la méthylation 1 (ainsi que pour la méthylation 2) est définie comme la quantité d'enzyme nécessaire pour incorporer 1 nmole de groupement méthyle dans la 4-amino-L-phénylalanine (ou dans la 4-méthylamino-L-phénylalanine).

7.2. Purification des protéines recombinantes de *S. pristinaespiralis* (sauvage et mutées) présentant les activités N-méthyl transférase SAM dépendante catalysant la méthylation de la 4-amino-L-phénylalanine en 4-méthylamino-L-phénylalanine et la méthylation de la 4-méthylamino-L-phénylalanine en 4-diméthylamino-L-phénylalanine.

Cet exemple illustre la purification d'une enzyme de *S. pristinaespiralis* SP92, intervenant dans la voie de biosynthèse de la pristinamycine IA, exprimée chez *E. coli* par clonage du gène papM modifié.

7.2.a. Purification de l'enzyme PapM mutant 49/66.

En utilisant le dosage décrit précédemment dans l'exemple 7.1, la purification de la N-méthyl transférase SAM dépendante est réalisée comme décrit ci-dessous en prenant soin de congeler et conserver à-70°C les fractions actives entre chaque étape si nécessaire.

Le culot de centrifugation d'une culture de *E. coli* BL21(DE3) : :pVRC721 (cf. exemple 6) surproduisant le variant 49/66 de PapM après induction par l'IPTG est lavé par un tampon pH 7.2 phosphate 100 mM, 1 mM PMSF, 5 mM EDTA, 5 mM EGTA, 0,5 M KCl, 10% v/v de glycérol. Deux grammes du culot lavé sont repris dans 20 ml de tampon pH 8 Tris-HCl 0,1M contenant 4 mM DTE, 5 mM benzamidine, 0,2 mM Pefabloc, 100 µg/l E-64, 2 mg/l leupeptine, 1 mM EDTA, 1 mM EGTA, 2 mg/l aprotinine, 20% glycérol et 2 mg/ml lysozyme. Ce tampon est maintenu à +4°C. La suspension ainsi obtenue est vigoureusement agitée à + 4°C. Après 30 min d'agitation, on ajoute 0,2 mg/ml désoxyribonucléase I et 5 mM $MgCl_2$. Après 90 min d'agitation, l'extrait est centrifugé pendant 1 heure à 50 000 g. Le surnageant est dessalé, en aliquotes de 2,5ml, par perméation de gel sur des colonnes de PD-10 (Pharmacia) équilibrée dans un tampon pH 6,8 bis-tris 20 mM contenant 4 mM DTE, 5 mM benzamidine, 0,2 mM Pefabloc, 100 μg/l E-64, 2 mg/l leupeptine 1 mM EDTA, 1 mM EGTA, 2 mg/l STI,. 2 mg/l aprotinine, 20% v/v glycérol. Une partie de l'éluat protéique est alors chromatographié (34 mg de protéine) sur une colonne MonoQ HR® 10/10 à un débit de 3 ml/min avec un gradient linéaire croissant de chlorure de sodium (0 à 0,3 M) dans un tampon pH 6,8 bis tris 20mM, contenant 4 mM DTE, 2mM benzamidine, 100 μg/l E-64 ,2 mg/l leupeptin 20% v/v glycérol. A la sortie de la colonne, les fractions sont complétées par 10% v/v de tampon pH 6,8 bis-tris 20 mM, contenant 4 mM DTE , 30 mM benzamidine, 2 mM Pefabloc, 100 μg/l E-64, 2 mg/l leupeptine, 5 mM EDTA, 5 mM EGTA, 10 mg/l STI, 10 mg/L aprotinine, 20% v/v glycérol. Dans ces conditions, les deux activités de méthylation ( 1 et 2) sont détectées de façon identique dans les fractions d'exclusion et les premières fractions d'élution. Ces fractions sont regroupées, concentrées par ultrafiltration sur Centriprep ® 10 puis sur Centricon ® 10. Ce concentrat est amené à 0,85 M de sulfate d'ammonium puis chromatographié (20 à 80 mg à chaque cycle) sur une colonne Phényl-Superose HR ®5/5 à un débit de 0,5 ml/min avec un gradient linéaire décroissant de sulfate d'ammonium (0,85 à 0 M) dans un tampon pH 6,8 bis tris 50mM, contenant 4 mM DTE, 2 mM benzamidine, 100 μg/l E-64, 2 mg/ml leupeptine, 1 mM EDTA, 1mM EGTA, 10 % v/v glycerol. A la sortie de la colonne, les fractions sont complétées par 10 % v/v de tampon pH 6,8 bis-tris 50 mM, contenant 4 mM DTE, 30 mM benzamidine, 2 mM Pefabloc, 100 μg/l E-64, 2 mg/l leupeptine, 1 mM EDTA, 1mM EGTA, 10 mg/l STI, 10 mg/l aprotinine, 10% glycérol. Dans ces conditions, les deux activités de méthylation (1 et 2) sont détectées de façon identique dans les fractions d'élution correspondant à environ 0,15 M sulfate d'ammonium.

Après cette étape, l'enzyme est pure. Elle présente en électrophorèse SDS-PAGE, une seule bande centrée à un poids moléculaire aux environs de 32000.

Tableau 4 : purification de l'enzyme 4-amino-L-phénylalanine (phényl N)-méthyltransférase mutant 49/66 à partir de la souche *E. coli* BL21 (DE3) :pVRC 721. Le facteur de purification est calculé d'après l'augmentation de l'activité spécifique des fractions au cours de la purification.

| Etape de purification | vol. (ml) | Proteines (mg) | Act.Spé. (unités[a]/mg) | Rendement (%) | Facteur de purification |
|---|---|---|---|---|---|
| Extrait brut | 4,3 | 33,7 | 3400 | - | - |
| PD 10 | 6 | 31,5 | 3400 | 93,5 | 1 |
| MonoQ HR10/10 | 17,5 | 3,0 | 19365 | 36,9 | 4,1 |
| Phényl-Superose | 1 | 0,145 | 28000 | 3,5 | 8,2 |
| [a] Il s'agit des unités d'activité enzymatique pour la méthylation 1 définies à l'exemple 7.1. | | | | | |

7.2.b. Purification des enzymes PapM sauvage, mutant 49 et mutant 66.

Les enzymes PapM wt, papM 49 et papM 66 sont purifiées à partir de cultures de E. coli DH5α transformées respectivement avec les plasmides pVRC706, pVRC711 et pVRC713 (cf. exemple 4) et induites par l' IPTG. Le protocole de purification utilisé est le même que celui qui est décrit à l'exemple 7.2.a

7.3. Mesure des constantes cinétiques Km et V max des méthylases purifiées PapM (sauvage et mutées)

Les conditions de dosage de l'activité de méthylation sont identiques à celles décrites dans l'exemple 7-1 à l'exception de la concentration en SAM de 2 mM dont le groupement méthyle est marqué radioactivement avec l'isotope 14 de l'atome de carbone (2 Ci/mol). Des gammes de concentration allant de 0,1 à 60 mM pour chacun des substrats 4-amino-L-phénylalanine pour la méthylation 1 et 4-méthylamino-L-phénylalanine pour la méthylation 2 ont été réalisées. Pour chaque concentration de chaque substrat, la vitesse de réaction est mesurée et exprimée en nmoles transformées par heure et par mg de proteines.

Les valeurs des constantes cinétiques sont déduites des courbes de Michaelis-Menten tracées à l'aide du logiciel Enzfiter.

Les valeurs obtenues pour l'enzyme native PapM sauvage et pour les formes mutantes PapM 49, PapM 66 et PapM 49/66 sont rassemblées dans le tableau ci-dessous.

Tableau 5

| Clone | PAPA | | MMPAPA | | Ratio Km2/Km1 | Ratio Vm1/Vm2 |
|---|---|---|---|---|---|---|
| | Km1 (mM) | Vm1 (nmoles/h/mg) | Km2 (mM) | Vm2 (nmoles/h/ mg) | | |
| wt | 0,5 | 112600 | 0,55 | 114000 | 1,1 | 0,99 |
| 49 | 12 | 10600 | 30 | 2820 | 2,5 | 3,8 |
| 66 | 0,57 | 45200 | 2,2 | 19700 | 3,9 | 2,3 |
| 49/66 | 9 | 28000 | 22 | 9100 | 2,5 | 3,1 |

Ces résultats montrent qu'il est possible d'obtenir par cette méthode une nouvelle enzyme montrant de nouvelles propriétés catalytiques par rapport à l'enzyme naturelle. Pour chaque forme mutante de l'enzyme, il a été déterminé l'efficacité enzymatique de chaque réaction de méthylation (méthylation 1 et méthylation 2). Cette efficacité est définie par le rapport Vm1/Km1 (méthylation 1) ou Vm2/Km2 (méthylation 2).

Puis, pour chaque forme, le ratio des efficacités enzymatiques (réaction de méthylation 1/ réaction de méthylation 2) a été déterminé. Ce ratio est égal à Vm1. Km$^2$ / Vm2. km1.

Le ratio obtenu pour l'enzyme Pap M sauvage est de 1,1.

Les mutants de Pap M ont été sélectionnés de telle façon que le ratio des efficacités enzymatiques soit supérieur à 2,5. Les meilleurs mutants ont montré des ratios supérieurs à 7 et de préférence voisins de 10.

Tableau 7

| | Km1 | Vm1 | Km2 | Vm2 | Vm1/Km1 | Vm2/Km2 | $\frac{Vm1.Km2}{Vm2\ Km1}$ |
|---|---|---|---|---|---|---|---|
| wt | 0.5 | 112600 | 0.55 | 114000 | 225200 | 207270 | 1.08 |
| | | | | | | | |
| 49 | 12 | 10600 | 30 | 2820 | 880 | 94 | 9.36 |
| 66 | 0.57 | 45200 | 2.2 | 19700 | 79300 | 8950 | 8.86 |
| 49/66 | 9 | 28000 | 22 | 9100 | 3110 | 410 | 7.58 |

7.4. Test de compétition entre les deux substrats 4-amino-L-phénylalanine et 4-méthylamino-L-phénylalanine.

Cet exemple illustre comment nous avons pu discriminer la méthylase la plus performante pour la production de PIB, c'est à dire celle qui catalyse le mieux la première réaction de méthylation de la p-amino-L-phénylalanine en p-méthyl-L-phénylalanine par rapport à la deuxième réaction de méthylation de la p-méthyl-L-aminophénylalanine en p-diméthylamino-L-phénylalanine.

Le ratio des vitesses de transformation des deux substrats compétitifs est donné par la formule:

$$V1/V2=(\ Kcat1/Km1\ )[S1]/(\ Kcat2/Km2\ )[S2]$$

Kcat étant le "turn over" de l'enzyme ou le nombre de moles de substrat méthylé par nombre de moles d'enzyme et par unité de temps.

Cette formule est valable pour toutes concentrations de S1 1 et de S2.

En fait, quelles que soient les concentrations de S1 et de S2 présentes, la réaction enzymatique est la somme de deux composantes, comme si elle était catalysée par deux enzymes séparées, l'une agissant sur S1 et inhibée compétitivement par S2, l'autre agissant sur S2 et inhibée compétitivement par S1

Toute modification du Km et/ou de la Vmax. de l'un des deux substrats se répercutera sur le taux de transformation de l'autre et entraînera une variation du ratio V1/V2.

On s'affranchit ainsi de la quantité de substrat et d'enzyme mise enjeu en prenant soin de ne pas transformer plus de 2% de chacun des substrats.

En utilisant les mêmes conditions de dosage de l'activité de méthylation que celles décrites dans l'exemple 7-1, à l'exception de la concentration de la SAM qui est de 2 mM et dont le groupement méthyle est marqué radioactivement avec l'isotope 14 de l'atome de carbone ( 2,5 Ci/mol ) et avec la présence simultanée des deux substrats aux concentrations en 4-amino-L-phénylalanine de 1 mM et en 4-méthylamino-L-phénylalanine de 2 mM, la vitesse de chacune des réactions a ainsi pu être déterminée.

Les essais sont réalisés simultanément sur les mutants décrits dans les exemples 4 à 6, et en utilisant les mêmes solutions de substrats.

Dans ces conditions, la méthylase doublement mutée 49/66 apparaît comme étant la plus sélective, elle catalyse la première réaction de méthylation 7 fois mieux que la deuxième, alors que l'enzyme native catalyse les deux réactions de manière similaire.

**EXEMPLE 8 : Construction de souches recombinantes de *S. pristinaespiralis* disruptées dans le gène papM**

Cet exemple illustre la construction de souches recombinantes de *S. pristinaespiralis* disruptées dans le gène papM. De telles souches constituent la première étape de la construction de souches recombinantes dont le gène papM sauvage est remplacé par un des allèles mutés obtenus et caractérisés comme il est décrit dans les exemples précédents. De plus ces souches produisent principalement un composé de la famille des PI, la PINH2 (Figure 3) qui n'est pas synthétisé naturellement par les souches possédant le gène sauvage papM. Ces souches représentent donc une voie d'accès à la PINH2 qu'il serait impossible d'isoler à partir de souches non disruptées dans le gène papM.

8.1. <u>Construction de souches recombinantes disruptées dans le gène papM</u>

La disruption du gène papM a été réalisée dans plusieurs souches de S. *pristinaespiralis :*

- la souche *S. pristinaespiralis* SP92 précédemment décrite qui produit un mélange de PI et PII (Blanc et al, 1994)

- la souche SP213 dérivée de SP92 après mutagénèse chimique. Cette souche produit spécifiquement des PI.

La stratégie de construction de souches recombinantes disruptées dans le gène papM est la même pour ces deux souches. Les résultats obtenus avec la souche SP213 sont détaillés dans la suite de cet exemple.

Cette construction a été réalisée à l'aide du plasmide pVRC1303 (cf figure 9) dérivé du vecteur suicide pDH5 capable de se répliquer uniquement chez E. *coli* et portant un gène de résistance s'exprimant chez *Streptomyces* (Hillemann et al., 1991).

8.1.1. <u>Construction du plasmide pVRC1303</u>

Le plasmide pVRC1303 a été construit après 4 étapes principales de clonage décrites sur la figure 9 à partir :

- du vecteur pDH5 portant une origine de réplication fonctionnelle chez E *coli,* le gène de résistance à l'ampicilline s'exprimant chez E. *coli* et le gène de résistance au thiostrepton et au nosiheptide s'exprimant chez *Streptomyces* (Hillemann et al., 1991)

- du plasmide pVRC409 (Blanc et al., 1994) pour préparer le fragment BsiWI-PstI de 2,5 kb portant une partie des gènes du cluster pristinamycine situés en aval du gène papM

- du plasmide pVRC900 (Blanc et al., 1994; Blanc et.. al, 1997) pour préparer le fragment PvuII-PvuII de 3,3 kb portant une partie des gènes du cluster pristinamycine situés en amont du gène papM

- des plasmides pHP45Ω-Km (Fellay et al., 1987) et pIJ702 (Hopwood et al., 1985) pour préparer, après une étape de clonage intermédiaire, le fragment EcoRI-EcoRI de 3,7 kb portant essentiellement le gène de résistance à la kanamycine s'exprimant chez *E. coli* et chez *Streptomyces.* Cette cassette Ω-Km portant le gène de résistance à la kanamycine est introduite entre les régions amont et aval du gène papM clonées dans le pVRC1303 et remplace le gène papM manquant (figure 9).

Toutes les étapes nécessaires à ces clonages (isolement d'ADN plasmidique, digestion d'ADN plasmidique par enzyme de restriction, remplissage d'extrémités 5' sortantes par traitement à l'enzyme Kleenow, migration et analyse de digestions enzymatiques par électrophorèse en gels d'agarose, préparation de fragments d'ADN après migration électrophorétique, réactions de ligation, transformation de *E. coli,* sélection et analyse des clones recombinants) ont été faites selon les protocoles standards décrits par Sambrook et al. (1989).

8.1.2. <u>construction de souches recombinantes de *S. pristinaespiralis* disruptées dans le gène papM par recombinaison homologue avec le plasmide pVRC1303</u>

Les souches recombinantes ont été isolées après transformation de la souche SP213 avec le plasmide pVRC1303.

La préparation des protoplastes de ces souches et leur transformation par la méthode utilisant le PEG ont été réalisées comme il est décrit dans Hopwood et al. (1985). Après transformation par 5 fois 1μg de pVRC1303, les protoplastes sont étalés sur milieu R2YE (Hopwood et al., 1985) et sélectionnés après une nuit de régénération par étalement d'une surcouche de 3 ml de milieu SNA contenant 10 μg/ml de kanamycine.

Sur les 5 transformations réalisées, 37 clones résistants à la kanamycine ont été isolés. Ces recombinants peuvent résulter :

- soit d'une intégration dans le chromosome du plasmide pVRC1303 à la suite d'une simple événement de recombinaison homologue entre les régions chromosomique et plasmidique situées en amont ou en aval du gène papM (cas de simple crossing over)

- soit d'un échange entre la cassette Ω-Km plasmidique portant le gène de résistance à la kanamycine et le gène chromosomique papM à la suite d'un double événement de recombinaison homologue entre les régions chromosomique et plasmidique situées en amont et en aval du gène papM (cas de double crossing over représenté sur la figure 10)

Dans ces deux cas, la cassette Ω-Km se trouve transférée sur le chromosome de la souche recombinante et lui confère une résistance à la kanamycine. Par contre ce n'est que dans le cas du simple crossing over que le gène présent dans le plasmide pVRC1303 (Figure 9) se trouve transféré sur le chromosome de la souche recombinante et lui confère une résistance au thiostrepton ou et au nosiheptide (Hillemann et al., 1991). Les deux cas de figure de crossing overs (simple ou double) peuvent donc être distingués par l'analyse du phénotype de résistance ou de sensibilité au nosiheptide.

Pour ce faire, les clones recombinants issus des 5 transformations par le pVRC1303 et sélectionnés pour leur résistance à 10 μg/ml de kanamycine comme il est décrit ci-dessus, ont été repiqués sous forme de patchs sur milieu HT (Hopwood et al., 1985) contenant 10 μg/ml de kanamycine puis testés sur milieu HT contenant 400 μg/ml de nosiheptide. Seuls les clones présentant le phénotype ($Kana^R$,$Nosi^s$) ont été sélectionnés car ils correspondent au cas de double crossing over recherché. Ces clones recombinants ont ensuite été purifiés de la façon suivante : on prépare un stock de spores de ces clones selon la méthode de Hopwood et al. (1955) après étalement et croissance sur milieu HT contenant 10 μg/ml de kanamycine que l'on dilue puis réétale sur le même milieu afin d'obtenir des colonies isolées. Ces colonies constituent des sous-clones purs des clones sélectionnés. On a ainsi analysé 2 sous-clones issus de 5 clones différents.

Afin de vérifier la structure chromosomique de ces sous-clones, on extrait leur ADN génomique après les avoir cultivé en milieu liquide YEME contenant 10 μg/ml de kanamycine selon la technique de lyse au lyzozyme et d'extraction au phénol/chloroforme décrite par Hopwood et al. (1985). On réalise plusieurs Southerns de l'ADN total de ces différents clones digéré par EcoRI et SphI que l'on hybride avec le plasmide pVRC1303 linéarisé par EcoRI et utilisé comme sonde après marquage par random priming obtenu avec le ($\alpha$-$^{32}P$) dCTP et le kit de marquage (Random primed DNA labelling kit) commercialisé par Amersham. Les techniques utilisées sont celles décrites par Sambrook et al. (1989). Les résultats d'hybridation montrent clairement que les sous-clones ont été obtenus par double crossing over entre le vecteur pVRC1303 et le chromosome de la souche SP213 décrit sur la figure 10. On vérifie notamment la présence dans ces sous-clones d'un fragment EcoRI de 9,9 kb hybridant avec la sonde qui n'est pas présent chez la souche sauvage SP213 pour laquelle la taille du fragment EcoRI hydridant est de 6.8 kb.

Un des clones ayant le génotype SP213ΔpapM :: Ω kanaR a été nommé SP216. Ce clone a été retenu pour l'analyse de sa production et pour poursuivre les constructions génétiques en vue d'obtenir des souches recombinantes dont le gène papM sauvage a été remplacé par un des allèles mutés ou doublement mutés de papM tels que décrits dans les exemples précédents.

<u>8.2. Production de pristinamycines par le mutant SP216.</u>

Cet exemple illustre comment il est déterminé que le mutant de *Spristinaespiralis* SP216 disrupté dans le gène papM produit principalement de la PI NH2 [4ξ-amino-dés(4ξ-diméthylamino) pristinamycine IA] dans les conditions standards de fermentation. La structure de la PINH2 est représentée sur la figure 2.

Le mutant SP216 ainsi que la souche sauvage témoin SP213 ont été cultivés en milieu de production liquide. La fermentation a été réalisée comme suit : 0,5 ml d'une suspension de spores de la souche précitée sont ajoutés en conditions stériles à 40 ml de milieu inoculum dans un erlen chicané de 300 ml. Le milieu inoculum est constitué de 10 g/l de Corn Steep, 15 g/l de saccharose, 10 g/l de $(NH_4)_2S0_4$, 1 g/l de $K_2HPO_4$, 3 g/l de NaCl, 0,2 g/l de $MgSO_4$-$7H_20$ et 1,25 g/l de $CaC0_3$. Le pH est ajusté à 6,9 par de la soude avant l'introduction du carbonate de calcium. Les erlens sont agités pendant 44 h à 27°C sur un agitateur rotatif à la vitesse de 325 rpm. On ajoute stérilement 2,5 ml de la culture précédente agée de 44 h à 30 ml de milieu de production dans un erlen de 300 ml. Le milieu de production est constitué de 25 g/l de farine de soja, 7,5 g/l d'amidon, 22,5 g/l de glucose, 3,5 g/l de levure fourragère, 0,5 g/l de sulfate de zinc et 6 g/l de carbonate de calcium. Le pH est ajusté à 6.0 avec de l'acide chlorhydrique avant l'introduction du carbonate de calcium.

Les erlens sont agités pendant 50 h à 27°C. Aussitôt la fermentation terminée, le volume de moût est mesuré et on ajoute deux volumes de phase mobile composée de 34 % d'acétonitrile et 66 % d'une solution de 0,1 M $KH_2PO_4$ (ajusté à pH 2.9 avec $H_3P0_4$ concentré) permettant l'extraction des pristinamycines. Après agitation, le tout est filtré, les pristinamycines sont contenues dans le surnageant. Elles sont alors dosées par CLHP en injectant 150 µl du surnageant de centrifugation sur une colonne Nucléosil 5-C8® de 4,6 x 150 mm éluée par un mélange de 40 % d'acétonitrile et de 60 % de tampon phosphate 0,1 M pH 2,9. Les pristinamycines sont détectées par absorption UV à 206 nm et éventuellement par leur émission de fluorescence (filtre 370 nm, excitation à 306 nm ). La production de pristinamycines est présentée dans les tableaux ci-dessous.

Tableau 8 : niveau de production (en mg/l) en PINH2 des souches SP213 et SP216

| Souche | PINH2 |
|---|---|
| SP213 (papM wt) | < 0,1 |
| SP216 (ΔpapM :: Ω kanaR) | 7 |

Ces résultats montrent que dans les conditions de fermentation réalisées, le mutant SP216 produit essentiellement de la PINH2, alors que le témoin SP213 produit une quantité standard de PI, dont la principale est la PIA.

**Exemple 9 : construction de souches recombinantes de *S. pristinaespiralis* comportant le gène papM allèle 49/66 ou le gène papM allèle 66.**

Cet exemple illustre la capacité des souches recombinantes de *S. pristinaespiralis* mutées dans le gène papM à produire sélectivement un composé de la famille des PI, la PIB (figure 2). La PIB est synthétisée naturellement par les souches possédant le gène sauvage papM mais à des taux très faibles d'environ 5% au maximum. Ces souches représentent donc une voie d'accès à la PIB produite ainsi en plus grande quantité et de manière très spécifique.

9.1. Construction de souches recombinantes mutées dans le gène papM

L'introduction du gène papM muté 49/66 a été réalisée dans la souche *S. pristinaespiralis* SP92 précédemment décrite qui produit un mélange de PI et PII (Blanc et al, 1994) et dans la souche SP213 dérivée de SP92 après mutagénèse chimique ; la souche SP213 produit spécifiquement des PI.

L'introduction du gène papM muté 66 a été réalisée dans la souche SP213 dérivée de SP92.

La stratégie de construction de souches recombinantes portant un des allèles mutés du gène papM est la même quelque soit le type de mutation de l'allèle du gène papM (49/66 ou 66) et quelque soit la souche choisie. Elle consiste à transformer les souches recombinantes dont le gène papM sauvage a été disrupté et remplacé par une cassette Ω-Km (cf exemple 8) par un plasmide suicide permettant l'échange de cette cassette par un des allèles mutés de papM.

A titre d'illustration, il est détaillé ci-après l'introduction du gène papM muté allèle 49/66 dans la souche SP216 (l'obtention de la souche SP216 est décrite dans l'exemple 8). Cette construction a été réalisée à l'aide du plasmide pVRC1306 (cf figure 11) dérivé du vecteur suicide pDH5 capable de se répliquer uniquement chez *E. coli* et portant un gène de résistance au thiostrepton ou au nosiheptide s'exprimant chez *Streptomyces* (Hillemann et al., 1991).

On a indiqué dans l'exemple 9.2, les résultats de production de pristinamycines obtenus avec plusieurs souches recombinantes dérivées de SP92 ou de SP213 et exprimant soit le gène muté papM 66, soit le gène muté papM49/66.

9.1.1. Construction du plasmide pVRC1306

Le plasmide pVRC1306 comportant portant le gène papM allèle 49/66 a été construit à partir du plasmide pVRC1303 portant une délétion du gène papM. cette construction est réalisée en trois étapes tel que décrit dans la figure 11. Pour ces clonages, les plasmides suivants ont été utilisés :

- le plasmide pBR322 est un vecteur de clonage dans *E. coli* qui a servi de vecteur intermédiaire pour transférer le fragment EcoRI de 8.9 kb du pVRC1303 décrit dans l'exemple 8.1.1 et contenant, entre autre, le gène papM délété par la cassette W-Km portant le gène de résistance à la kanamycine. Le clonage s'est fait au niveau du site EcoRI du pBR322 et le plasmide obtenu a été nommé pVRC1304.
- le plasmide pVRC 721 décrit dans l'exemple 6 (figure 6) a servi à préparer l'insert MluI/AgeI de 1 kb contenant le gène papM allèle 49/66. Cet insert a ensuite été échangé avec le fragment MluI/AgeI de 4 kb du pVRC1304 et le plasmide obtenu a été nommé pVRC1305.
- le plasmide pVRC1303 a permis d'échanger le fragment EcoRI de 8.9 kb avec le fragment EcoRI de 5.83 kb du pVRC1305 contenant le gène papM allèle 49/66.

Le plasmide final pVRC1306 (figure 11) comporte donc le gène papM allèle 49/66 ainsi qu'une partie amont et aval de ce gène, l'ensemble étant cloné dans le vecteur pDH5 décrit dans l'exemple 8.1.1

9.1.2. Construction de souches recombinantes de *S. pristinaespiralis* mutées dans le gène papM allèle 49/66 par recombinaison homologue avec le plasmide pVRC1306

Les souches recombinantes ont été isolées après transformation de la souche SP216 décrite dans l'exemple 8 avec le plasmide pVRC1306. Pour cela deux étapes ont été nécessaires. La première étape a consisté à sélectionner par analyse du phénotype ($Kana^R$,$Nosi^R$) une souche issue d'un simple évènement de recombinaison homologue entre les régions chromosomique et plasmidique situées en amont du gène papM (figure 12). La deuxième étape a consisté à sélectionner, à partir de la souche ($Kana^R$,$Nosi^R$), le deuxième évènement de recombinaison homologue par analyse du phénotype ($Kana^S$,$Nosi^S$) des clones obtenus (figure 13).
La réalisation de ces deux étapes est décrite ci-après.
La préparation des protoplastes de ces souches et leur transformation par la méthode utilisant le PEG ont été réalisées comme il est décrit dans Hopwood et al. (1985). Dix transformations indépendantes ont été faites avec pour chacune, 1 $\mu$g de pVRC1306. Les protoplastes sont étalés sur milieu R2YE (Hopwood et al., 1985) et sélectionnés après une nuit de régénération par étalement d'une surcouche de 3 ml de milieu SNA contenant 400 $\mu$g/ml de nosiheptide.
Sur les 10 transformations réalisées, 7 clones résistants au nosiheptide (Hillemann et al., 1991) ont été isolés. Ces recombinants résultent d'une intégration dans le chromosome du plasmide pVRC1306 à la suite d'un simple évènement de recombinaison homologue entre les régions chromosomique et plasmidique situées en amont du gène papM, ce qui confère à ces clones une double résistance, à la fois à la kanamycine (du fait de la souche transformée) et au thiostrepton (du fait de la recombinaison du plasmide). Les 7 clones recombinants ont donc été repiqués sur milieu HT avec 10 $\mu$g/ml de kanamycine et sur milieu HT avec 400 $\mu$g/ml de nosiheptide. La totalité des clones présente le phénotype attendu ($Kana^R$,$Nosi^R$). Ces clones ont été purifiés deux fois selon la méthode suivante : un stock de spores de ces clones a est préparé selon la méthode de Hopwood et al. (1985) et des colonies isolées sont obtenues après étalement de différentes dilutions du stock sur milieu HT contenant 400 $\mu$g/ml de nosiheptide. Ces colonies constituent des sous-clones purs des clones sélectionnés. La structure chromosomique de deux sous-clones issus de deux clones a été analysée par Southem Blot.
L'ADN génomique a été extrait après culture en milieu liquide YEME contenant 4 $\mu$g/ml de nosiheptide. L'ADN a été extrait par traitement au lyzozyme et extraction au phénol/chloroforme selon la technique décrite par Hopwood et al. (1985). Plusieurs Southerns ont été réalisés avec l'ADN total de ces différents sous clones digérés par les enzymes de restriction EcoRI et AseI. Le fragment de 8.9 kb issu du pVRC1303 digéré par EcoRI a été utilisé comme sonde après marquage par random priming obtenu avec le ($\alpha$ -32P) dCTP et le kit de marquage (Random primed DNA labeling kit) commercialisé par Amersham. Les techniques utilisées sont celles décrites par Sambrook et al. (1989). Les résultats d'hybridation (bandes correspondant à 6.83, 3.42, 3.125 et 2.06 kb) ont montré clairement que les sous-clones ont été obtenus par simple crossing over entre le vecteur pVRC1306 et le chromosome de la souche SP216. L'ADN de la souche SP216 présente les bandes correspondant à 4.42, 3.125 et 2.06 kb mais ne présente pas la bande correspondant à celle de 6.83 kb.
Un de ces clones a été nommé SP216::pVRC1306.
La deuxième étape consiste à sélectionner un deuxième évènement de recombinaison homologue entre les régions chromosomique et plasmidique situées en aval du gène papM à partir de la souche SP216::pVRC1306 (cf figure 13). Cette étape conduit à la sélection de clones recombinants ayant échangé le gène papM délété par la cassette Ω-Km avec le gène papM alléle 49/66 ; le phénotype attendu des clones recombinants est ($Kana^S$,$Nosi^S$).

A partir du clone SP216::pVRC1306, 2 cycles de croissance ont été réalisés à 30 °C sur un agitateur rotatif à la vitesse de 325 rpm afin de favoriser le deuxième évènement de recombinaison. Le premier cycle a été fait avec 200 μl d'un stock de spores de SP216::pVRC1306 dans 10 ml de milieu YEME (conditions de culture : 30 °C, 280 rpm, 72 heures), le deuxième cycle a été fait avec 800 μl de la suspension du premier cycle dans 40 ml de milieu YEME (conditions de culture : 30 °C, 280 rpm, 72 heures). La deuxième culture a été centrifugée 15 mn à 3500 tr/mn, le culot a été repris dans 600 μl d'eau + Tween 0.01% et cette suspension a été étalée sur milieu HT pour obtenir des tapis de spores. Afin de purifier les clones recombinants issus de cette culture liquide, les spores ont été récoltées et des suspensions diluées de ces spores ont été étalées sur milieu HT pour obtenir des colonies isolées.

Pour sélectionner des clones ayant le phénotype désiré ($Kana^s$,$Nosi^s$), 1000 colonies isolées ont été repiquées et screenées. Ces colonies ont été repiquées automatiquement sur milieu HT sans antibiotique et sur milieu HT avec 10 μg/ml de kanamycine. Cette première étape a permis de sélectionner 18 clones recombinants $Kana^s$. A partir du milieu HT sans antibiotique, ces 18 clones ont été repiqués manuellement sur milieu HT sans antibiotique, sur milieu HT avec 10 μg/ml de kanamycine et sur milieu HT avec 400 μg/ml de nosiheptide. Trois clones recombinants ayant le phénotype ($Kana^s$,$Nosi^s$) ont été sélectionnés comme étant potentiellement issus d'événement de double crossing over. Ces clones ont été purifiés, un stock de spores a été fait à partir de tapis de spores ainsi qu'une analyse génomique; les méthodes utilisées étant décrites ci-dessus.

L'analyse chromosomique des sous-clones recombinants est faite en deux étapes : la première étape consiste a vérifier que le gène papM allèle 49/66 est présent et que les régions chromosomiques en amont et en aval de ce gène n'ont pas été modifiées. La deuxième étape consiste à vérifier l'absence de fragment d'ADN correspondant à la cassette W-Km et au plasmide pDH5.

Pour la première étape, des préparation d'ADN génomique des clones recombinants et des analyses d'ADN total en Southem Blot ont été réalisés selon les méthodes citées plus haut. L'ADN total a été digéré par les enzymes de restriction EcoRI et AseI. Le plasmide pVRC721 a été digéré par MluI (insert de 1.7 kb contenant le gène papM allèle 49/66). Ce fragment marqué a servi de sonde qui hybride avec un fragment correspondant à une bande de 6.83 kb.

D'autre part l'ADN total a été digéré par SalI. Des fragments marqués issus du pVRC409 et du pVRC803 (contenant les régions amont et aval du gène papM de la souche SP213, Blanc et al., 1994; Blanc et al., 1997) ont servis de sonde. Ces fragments ont permis de révéler, après hybridation, 7 bandes (2.45, 0.190, 2.150, 0.725,1.55,0.4 et 0.55 kb).

La deuxième étape a consisté à vérifier qu'il n'y avait plus de fragment de DNA correspondant à la cassette Ω -Km ou au plasmide pDH5. L'ADN total a été digéré par EcoRI et AseI. Le fragment HindIII du pHP45 Ω -Km (Fellay et all., 1987) de 2.2 kb (cassette Ω -Km) ainsi que le pDH5 ont été utilisés comme sonde.

Les trois clones recombinants présentent un profil chromosomique (figure13) qui confirme la présence du gène papM allèle 49/66 et l'absence du gène de résistance à la kanamycine ainsi que du fragment correspondant au plasmide pDH5. Ces éléments indiquent que l'environnement génomique du gène papM allèle 49/66 est intact. Un clone a été retenu et a été nommé souche SP217.

De la même façon d'autres souches de *S. pristinaespiralis* intégrant le gène papM muté allèle 49/66 ou le gène papM allèle 66 ont été construites. Il s'agit des souches :

- SP101 issue de la transformation de la souche SP 92 avec le plasmide pVRC1306 portant le gène papM muté allèle 49/66

- SP218 issue de la transformation de la souche SP216 avec un plasmide équivalent au pVRC1306 mais dans lequel le gène papM muté allèle 49/66 a été remplacé par le gène papM muté allèle 66.

9.2. Production de pristinamycines par les souches *P. pristinaespiralis* SP213, SP217, SP218, SP100 et SP101

Cet exemple montre la production de pristinamycines avec les différents mutants. Il illustre notamment l'augmentation de production de PIB des mutants papM muté allèle 49/66 (souche SP217) et des mutants papM allèle 66 (souche SP218) par rapport à la souche témoin papM wt (souche SP213). L'effet du gène papM muté allèle 49/66 (souche SP101) a également été comparé par rapport à la souche témoin papM wt (souche SP92).

Les techniques de production , d'extraction et de dosage des pristinamycines sont identiques à celles décrites dans l'exemple 8.2.

Les niveaux de production de différentes pristinamycines exprimés par rapport au taux de PIA des souches témoins (SP213 et SP92) sont présentés dans le tableau ci-dessous.

Tableau 9 : production de pristinamycines PI pour différentes souches S. pristinaespiralis. Les valeurs de PI produites sont données en mg/litre

| Souche | PIA | PIB | PINH2 | Autres PI |
|---|---|---|---|---|
| SP213 (papM wt) | 474 | 39 | 0 | 25 |
| SP217 (papM 49/66) | 0 | 162 | 57 | 19 |
| SP218 (papM 66) | 19 | 271 | 14 | 32 |
| | | | | |
| Souche | PIA | PIB | | |
| SP92 (papM wt) | 100 | 3 | | |
| SP101(papM66) | 0 | 11 | | |

Ces résultats confirment que les gènes exprimant les variants du polypeptide papM selon l'invention confèrent aux souches de *S. pristinaespiralis* une excellente sélectivité pour la production de PIB.

## Revendications

**1.** Polypeptide PapM de bactéries du genre Streptomyces, **caractérisé en ce qu'**il dérive de la séquence du polypeptide PapM sauvage de S. pristinaespiralis par remplacement d'un ou plusieurs acides aminés choisis parmi

a) le résidu Gly 249 du polypeptide PapM de S. pristinaespiralis,
b) le résidu Thr 192 du polypeptide Pap M de S. pristinaespiralis.

**2.** Polypeptide selon la revendication 1, **caractérisé en ce qu'**il est dérivé de la séquence polypeptidique SEQ ID N°2 et **en ce qu'**il présente au moins une substitution en position 249 d'une Glycine par une Sérine.

**3.** Polypeptide selon la revendication 1, **caractérisé en ce qu'**il est dérivé de la séquence polypeptidique SEQ ID N°2 et **en ce qu'**il présente au moins une substitution en position 192 d'une Thréonine par une Isoleucine.

**4.** Polypeptide selon la revendication 1, **caractérisé en ce qu'**il est dérivé de la SEQ ID N°2 et **en ce qu'**il présente au moins une substitution en position 249 d'une Glycine par une Sérine et une substitution en position 192 d'une Thréonine par une Isoleucine.

**5.** Acide nucléique codant pour un polypeptide selon l'une des revendications 1 à 4.

**6.** Acide nucléique selon la revendication 5, **caractérisé en ce qu'**il dérive de la séquence nucléotidique présentée en SEQ ID N°1 ou les séquences dérivées en raison de la dégénérescence du code génétique.

**7.** Acide nucléique selon la revendication 6, **caractérisé en ce qu'**il comprend au moins une mutation faux-sens en amont du motif NPPY situé aux positions 193 à 196.

**8.** Acide nucléique selon la revendication 7, **caractérisé en ce que** la mutation faux-sens entraîne un changement non-conservatif d'acide aminé.

**9.** Acide nucléique selon la revendication 8, **caractérisé en ce qu'**il comprend au moins une substitution d'une cytosine en position 658 par une thymine (C658T).

**10.** Acide nucléique selon la revendication 6, **caractérisé en ce qu'**il comprend au moins une substitution d'une guanine en position 828 par une adénine (G828A).

**11.** Acide nucléique selon la revendication 8, **caractérisé en ce qu'**il comprend au moins une substitution d'une guanine en position 828 par une adénine (G828A) et au moins une substitution d'une cytosine en position 658 par une thymine (C658T).

**12.** ADN recombinant comprenant un acide nucléique selon l'une des revendications 5 à 11.

**13.** Vecteur d'expression à réplication autonome et/ou intégratif **caractérisé en ce qu'**il comprend un acide nucléique selon l'une des revendications 5 à 11 ou un ADN recombinant selon la revendication 12.

**14.** Vecteur selon la revendication 13, **caractérisé en ce qu'**il correspond au vecteur pVRC1306 tel que représenté sur la figure 11.

**15.** Cellule hôte contenant un acide nucléique selon l'une des revendications 5 à 11 et/ou un ADN recombinant selon la revendication 12 et/ou un vecteur d'expression selon l'une des revendications 13 ou 14.

**16.** Cellule hôte selon la revendication 15, **caractérisée en ce qu'**elle est choisie parmi *E.coli*, *S. pristinaespiralis, Streptomyces olivaceus* ATCC12019, *Streptomyces ostreogriseus* ATCC27455, *Streptomyces mitakaensis* ATCC15297, *Streptomyces loïdensis* ATCC11415, *Streptomyces graminofaciens, Streptomyces diastaticus*

**17.** Procédé de production d'un polypeptide selon l'une des revendications 1 à 4, **caractérisé en ce que** l'on cultive une cellule hôte selon les revendications 15 ou 16 et on récupère le polypeptide produit.

**18.** Utilisation d'une cellule hôte selon les revendications 15 ou 16, exprimant un polypeptide selon une des revendications 1 à 4 dans une réaction de bioconversion visant la conversion d'amines aromatiques en amines monométhylées.

**19.** Utilisation d'un acide nucléique selon l'une des revendications 5 à 11, pour modifier la proportion des différentes composantes B des Streptogramines dans une souche productrice de Streptogramines.

**20.** Utilisation selon la revendication 19 pour la production de PIB **caractérisée en ce que** l'acide nucléique utilisé est un acide nucléique codant pour un polypeptide selon l'une des revendications 1 à 4 et la souche productrice de Streptogramine est une souche de *S. pristinaespiralis.*

**21.** Utilisation selon la revendication 19 pour la production de PIA **caractérisée en ce que** l'acide nucléique utilisé est un acide nucléique codant pour un polypeptide selon la revendication 8 et la souche productrice de Streptogramine est une souche de *S. pristinaespiralis.*

**22.** Procédé de production d'une ou plusieurs composantes B des Streptogramines **caractérisé en ce que** :

- on inactive dans une souche productrice de Streptogramines ou potentiellement productrice de Streptogramines le gène PapM et on introduit une ou plusieurs copies d'un acide nucléique codant pour un polypeptide PapM tel que défini selon l'une des revendications 1 à 4 ,
- on cultive ladite souche dans des conditions de production des Streptogramines, et,
- on récupère la ou les composantes B des Streptogramines produites.

**23.** Procédé selon la revendication 22, **caractérisé en ce que** la souche productrice de Streptogramines est une souche productrice de Pristinamycines dérivée de *S. pristinaespiralis* et de préférence dérivée de la souche *S. pristinaespiralis* SP92 ou des souches *Streptomyces olivaceus* ATCC12019, *Streptomyces ostreogriseus* ATCC27455, *Streptomyces mitakaensis* ATCC15297, *Streptomyces loïdensis* ATCC11415, *Streptomyces graminofaciens, Streptomyces diastaticus.*

**24.** Procédé selon l'une des revendications 22 ou 23, **caractérisé en ce que** la souche productrice de Streptogramines est une souche qui produit une quantité réduite ou non détectable de composantes A des Streptogramines

**25.** Procédé selon l'une des revendications 22 à 24, **caractérisé en ce que** l'introduction de l'acide nucléique codant pour un polypeptide PapM selon l'une des revendications 1 à 4 se fait en remplacement de la forme sauvage du gène PapM.

**26.** Procédé selon la revendication 25 utile pour la production de PIB **caractérisé en ce que** la forme sauvage du gène PapM est remplacée par un acide nucléique codant pour un polypeptide selon l'une des revendications 1 à 4.

**27.** Procédé selon l'une des revendications 22 à 25 utile pour la production de PINH2 **caractérisé en ce que** la forme sauvage du gène PapM est remplacée par un acide nucléique codant une forme inactive du polypeptide PapM.

**28.** Procédé selon la revendication 26, **caractérisé en ce que** la souche utilisée comprend un gène PapM présentant la double mutation (G828A) (C658T).

**29.** Procédé selon la revendication 26, **caractérisé en ce que** la souche utilisée comprend un gène PapM présentant la mutation (G828A).

**30.** Procédé selon la revendication 26, **caractérisé en ce que** la souche utilisée comprend un gène PapM présentant la mutation (C658T).

**31.** Souche mutante de *S. pristinaespiralis* **caractérisée en ce qu'**elle comprend un acide nucléique selon l'une des revendications 5 à 11.

## Claims

**1.** PapM polypeptide of bacteria of the Streptomyces genus, **characterized in that** it derives from the sequence of the wild-type PapM polypeptide of S. pristinaespiralis by replacement of one or more amino acids chosen from

a) the residue Gly 249 of the Pap M polypeptide of S. pristinaespiralis
b) the residue Thr 192 of the Pap M polypeptide of S. pristinaespiralis.

**2.** Polypeptide according to Claim 1, **characterized in that** it derives from the polypeptide sequence SEQ ID NO. 2 and **in that** it exhibits at least one substitution, at position 249, of a glycine with a serine.

**3.** Polypeptide according to Claim 1, **characterized in that** it derives from the polypeptide sequence SEQ ID NO. 2 and **in that** it exhibits at least one substitution, at position 192, of a threonine with an isoleucine.

4. Polypeptide according to Claim 1, **characterized in that** it derives from SEQ ID NO. 2 and **in that** it exhibits at least one substitution, at position 249, of a glycine with a serine and a substitution, at position 192, of a threonine with an isoleucine.

5. Nucleic acid encoding a polypeptide according to one of Claims 1 to 4.

6. Nucleic acid according to Claim 5, **characterized in that** it derives from the nucleotide sequence presented in SEQ ID NO. 1 or the sequences derived due to the degeneracy of the genetic code.

7. Nucleic acid according to Claim 6, **characterized in that** it comprises at least one missense mutation upstream of the NPPY motif located at positions 193 to 196.

8. Nucleic acid according to Claim 7, **characterized in that** the missense mutation leads to a non-conservative amino acid change.

9. Nucleic acid according to Claim 8, **characterized in that** it comprises at least one substitution of a cytosine at position 658 with a thymine (C658T).

10. Nucleic acid according to Claim 6, **characterized in that** it comprises at least one substitution of a guanine at position 828 with an adenine (G828A).

11. Nucleic acid according to Claim 8, **characterized in that** it comprises at least one substitution of a guanine at position 828 with an adenine (G828A) and at least one substitution of a cytosine at position 658 with a thymine (C658T).

12. Recombinant DNA comprising a nucleic acid according to one of Claims 5 to 11.

13. Expression vector which replicates autonomously and/or which integrates, **characterized in that** it comprises a nucleic acid according to one of Claims 5 to 11 or a recombinant DNA according to Claim 12.

14. Vector according to Claim 13, **characterized in that** it corresponds to the vector pVRC1306 as represented in Figure 11.

15. Host cell containing a nucleic acid according to one of Claims 5 to 11 and/or a recombinant DNA according to Claim 12 and/or an expression vector according to either of Claims 13 and 14.

16. Host cell according to Claim 15, **characterized in that** it is chosen from *E. coli, S. pristinaespiralis, Streptomyces olivaceus* ATCC12019, *Streptomyces ostreogriseus* ATCC27455, *Streptomyces mitakaensis* ATCC15297, *Streptomyces loïdensis* ATCC11415, *Streptomyces graminofaciens* and *Streptomyces diastaticus.*

17. Method for producing a polypeptide according to one of Claims 1 to 4, **characterized in that** a host cell according to Claim 15 or 16 is cultured and the polypeptide produced is recovered.

18. Use of a host cell according to Claim 15 or 16, expressing a polypeptide according to one of Claims 1 to 4, in a bioconversion reaction aiming to convert aromatic amines to monomethylated amines.

19. Use of a nucleic acid according to one of claims 5 to 11, for modifying the proportion of the various B components of Streptogramins in a Streptogramin-producing strain.

20. Use according to Claim 19, for producing PIB, **characterized in that** the nucleic acid used is a nucleic acid encoding a polypeptide according to one of claims 1 to 4 and the Streptogramin-producing strain is a strain of *S. pristinaespiralis.*

21. Use according to Claim 19, for producing PIA, **characterized in that** the nucleic acid used is a nucleic acid encoding a polypeptide according to claim 8 and the Streptogramin-producing strain is a strain of *S. pristinaespiralis.*

22. Method for producing one or more B components of Streptogramins, **characterized in that**:

- the PapM gene is inactivated in a Streptogramin-producing or potentially Streptogramin-producing strain, and one or more copies of a nucleic acid encoding a PapM polypeptide as defined according to one of Claims 1 to 4 are introduced,
- said strain is cultured under conditions for producing Streptogramins, and
- the B component(s) of the Streptogramins produced is (are) recovered.

**23.** Method according to Claim 22, **characterized in that** the Streptogramin-producing strain is a Pristinamycin-producing strain derived from *S. pristinaespiralis,* and preferably derived from the strain *S. pristinaespiralis* SP92 or from the strains *Streptomyces olivaceus* ATCC12019, *Streptomyces ostreogriseus* ATCC27455, *Streptomyces mitakaensis* ATCC15297, *Streptomyces loïdensis* ATCC 11415, *Streptomyces graminofaciens* and *Streptomyces diastaticus.*

**24.** Method according to either of Claims 22 and 23, **characterized in that** the Streptogramin-producing strain is a strain which produces a small or undetectable amount of A components of Streptogramins.

**25.** Method according to one of Claims 22 to 24, **characterized in that** the introduction of the nucleic acid encoding a PapM polypeptide according to one of Claims 1 to 4 is carried out by replacement of the wild-type form of the PapM gene.

**26.** Method according to Claim 25, which is of use for producing PIB, **characterized in that** the wild-type form of the PapM gene is replaced with a nucleic acid encoding a polypeptide according to one of Claims 1 to 4.

**27.** Method according to one of Claims 22 to 25, which is of use for producing PINH2, **characterized in that** the wild-type form of the PapM gene is replaced with a nucleic acid encoding an inactive form of the PapM polypeptide.

**28.** Method according to Claim 26, **characterized in that** the strain used comprises a PapM gene exhibiting the double mutation (G828A) (C658T).

**29.** Method according to Claim 26, **characterized in that** the strain used comprises a PapM gene exhibiting the mutation (G828A).

**30.** Method according to Claim 26, **characterized in that** the strain used comprises a PapM gene exhibiting the mutation (C658T).

**31.** Mutant strain of *S. pristinaespiralis,* **characterized in that** it comprises a nucleic acid according to one of Claims 5 to 11.

## Patentansprüche

**1.** PapM-Polypeptid von Bakterien der Gattung Streptomyces, **dadurch gekennzeichnet, daß** es sich von der Sequenz des Wildtyp-PapM-Polypeptids von S. pristinaespiralis durch Ersatz von einer oder mehreren Aminosäuren ausgewählt aus der Reihe

a) Gly249-Rest des PapM-Polypeptids von S. pristinaespiralis,
b) Thr192-Rest des PapM-Polypeptids von S. pristinaespiralis

ableitet.

**2.** Polypeptid nach Anspruch 1, **dadurch gekennzeichnet, daß** es sich von der Polypeptidsequenz SEQ ID NO. 2 ableitet und daß es mindestens eine Substitution eines Glycins durch ein Serin in Position 249 aufweist.

**3.** Polypeptid nach Anspruch 1, **dadurch gekennzeichnet, daß** es sich von der Polypeptidsequenz SEQ ID NO. 2 ableitet und daß es mindestens eine Substitution eines Threonins durch ein Isoleucin in Position 192 aufweist.

**4.** Polypeptid nach Anspruch 1, **dadurch gekennzeichnet, daß** es sich von der Polypeptidsequenz SEQ ID NO. 2 ableitet und daß es mindestens eine Substitution eines Glycins durch ein Serin in Position 249 und eine Substitution eines Threonins durch ein Isoleucin in Position 192 aufweist.

**5.** Nukleinsäure, die für ein Polypeptid nach einem der Ansprüche 1 bis 4 codiert.

**6.** Nukleinsäure nach Anspruch 5, **dadurch gekennzeichnet, daß** sie sich von der Nukleotidsequenz gemäß SEQ ID NO. 1 oder den aufgrund der Degeneration des genetischen Codes abgeleiteten Sequenzen ableitet.

**7.** Nukleinsäure nach Anspruch 6, **dadurch gekennzeichnet, daß** sie mindestens eine "missense"-Mutation stromaufwärts des in Position 193 bis 196 gelegenen NPPY-Motivs aufweist.

**8.** Nukleinsäure nach Anspruch 7, **dadurch gekennzeichnet, daß** die "missense"-Mutation zu einem nichtkonservativen Aminosäureaustausch führt.

**9.** Nukleinsäure nach Anspruch 8, **dadurch gekennzeichnet, daß** sie mindestens eine Substitution eines Cytosins in Position 658 durch ein Thymin (C658T) umfaßt.

**10.** Nukleinsäure nach Anspruch 6, **dadurch gekennzeichnet, daß** sie mindestens eine Substitution eines Guanins in Position 828 durch ein Adenin (G828A) umfaßt.

**11.** Nukleinsäure nach Anspruch 8, **dadurch gekennzeichnet, daß** sie mindestens eine Substitution eines Guanins in Position 828 durch ein Adenin (G828A) und mindestens eine Substitution eines Cytosins in Position 658 durch ein Thymin (C658T) umfaßt.

**12.** Rekombinante DNA, die eine Nukleinsäure nach einem der Ansprüche 5 bis 11 umfaßt.

**13.** Autonom replizierender und/oder integrativer Expressionsvektor, **dadurch gekennzeichnet, daß** er eine Nukleinsäure nach einem der Ansprüche 5 bis 11 oder eine rekombinante DNA nach Anspruch 12 umfaßt.

**14.** Vektor nach Anspruch 13, **dadurch gekennzeichnet, daß** er dem Vektor pVRC1306 gemäß Abbildung 11 entspricht.

**15.** Wirtszelle, die eine Nukleinsäure nach einem der Ansprüche 5 bis 11 und/oder eine rekombinante DNA nach Anspruch 12 und/oder einen Expressionsvektor nach einem der Ansprüche 13 oder 14 enthält.

**16.** Wirtszelle nach Anspruch 15, **dadurch gekennzeichnet, daß** sie aus der Reihe *E. coli, S. pristinaespiralis, Streptomyces olivaceus* ATCC12019, *Streptomyces ostreogriseus* ATCC27455, *Streptomyces mitakaensis* ATCC15297, Streptomyces *loïdensis* ATCC11415, *Streptomyces graminofaciens, Streptomyces diastaticus* stammt.

**17.** Verfahren zur Herstellung eines Polypeptids nach einem der Ansprüche 1 bis 4, **dadurch gekennzeichnet, daß** man eine Wirtszelle nach den Ansprüchen 15 oder 16 kultiviert und das produzierte Polypeptid gewinnt.

**18.** Verwendung einer Wirtszelle nach Anspruch 15 oder 16, die ein Polypeptid nach einem der Ansprüche 1 bis 4 exprimiert, in einer Biokonversionsreaktion zur Konversion von aromatischen Aminen in einfach methylierte Amine.

**19.** Verwendung einer Nukleinsäure nach einem der Ansprüche 5 bis 11 zum Modifizieren des Anteils der unterschiedlichen Streptogramin-Komponenten B in einem Streptogramin-Produzentenstamm.

**20.** Verwendung nach Anspruch 19 zur Produktion von PIB, **dadurch gekennzeichnet, daß** es sich bei der verwendeten Nukleinsäure um eine Nukleinsäure handelt, die für ein Polypeptid nach einem der Ansprüche 1 bis 4 codiert, und daß es sich bei dem Streptogramin-Produzentenstamm um einen Stamm von *S. pristinaespiralis* handelt.

**21.** Verwendung nach Anspruch 19 zur Produktion von PIA, **dadurch gekennzeichnet, daß** es sich bei der verwendeten Nukleinsäure um eine Nukleinsäure handelt, die für ein Polypeptid nach Anspruch 8 codiert, und das es sich bei dem Streptogramin-Produzentenstamm um einen Stamm von *S. pristinaespiralis* handelt.

**22.** Verfahren zur Herstellung von einer oder mehreren Streptogramin-Komponenten B, **dadurch gekennzeichnet, daß** man:

- das PapM-Gen in einem Streptogramin-Produzentenstamm oder potentiellem Streptogramin-Produzentenstamm inaktiviert und eine oder mehrere Kopien einer Nukleinsäure, die für ein PapM-Polypeptid gemäß einem der Ansprüche 1 bis 4 codiert, einführt,
- diesen Stamm unter Streptograminproduktionsbedingungen kultiviert und
- die produzierte(n) Streptogramin-Komponente(n) B gewinnt.

**23.** Verfahren nach Anspruch 22, **dadurch gekennzeichnet, daß** es sich bei dem Streptogramin-Produzentenstamm um einen Pristinamycin-Produzentenstamm handelt, der sich von S. *pristinaespiralis,* vorzugsweise vom Stamm S. *pristinaespiralis* SP92 oder von den Stämmen *Streptomyces olivaceus* ATCC12019, *Streptomyces ostreogriseus* ATCC27455, *Streptomyces mitakaensis* ATCC15297, *Streptomyces loïdensis* ATCC11415, *Streptomyces graminofaciens, Streptomyces diastaticus,* ableitet.

**24.** Verfahren nach einem der Ansprüche 22 oder 23, **dadurch gekennzeichnet, daß** es sich bei dem Streptogramin-Produzentenstamm um einen Stamm handelt, der eine verringerte oder nicht nachweisbare Menge an Streptogramin-Komponenten A produziert.

**25.** Verfahren nach einem der Ansprüche 22 bis 24, **dadurch gekennzeichnet, daß** die Einführung der Nukleinsäure, die für ein PapM-Polypeptid nach einem der Ansprüche 1 bis 4 codiert, als Ersatz der Wildform des PapM-Gens erfolgt.

**26.** Verfahren nach Anspruch 25 für die Produktion von PIB, **dadurch gekennzeichnet, daß** die Wildform des PapM-Gens durch eine Nukleinsäure, die für ein Polypeptid nach einem der Ansprüche 1 bis 4 codiert, ersetzt wird.

**27.** Verfahren nach einem der Ansprüche 22 bis 25 für die Produktion von PINH2, **dadurch gekennzeichnet, daß** der Wildtyp des PapM-Gens durch eine Nukleinsäure, die für eine inaktive Form des PapM-Polypeptids codiert, ersetzt wird.

**28.** Verfahren nach Anspruch 26, **dadurch gekennzeichnet, daß** der verwendete Stamm ein PapM-Gen mit der Doppelmutation (G828A) (C658T) umfaßt.

**29.** Verfahren nach Anspruch 26, **dadurch gekennzeichnet, daß** der verwendete Stamm ein PapM-Gen mit der Mutation (G828A) umfaßt.

**30.** Verfahren nach Anspruch 26, **dadurch gekennzeichnet, daß** der verwendete Stamm ein PapM-Gen mit der Mutation (C658T) umfaßt.

**31.** Mutantenstamm von *S. pristinaespiralis,* **dadurch gekennzeichnet, daß** er eine Nukleinsäure nach einem der Ansprüche 5 bis 11 umfaßt.

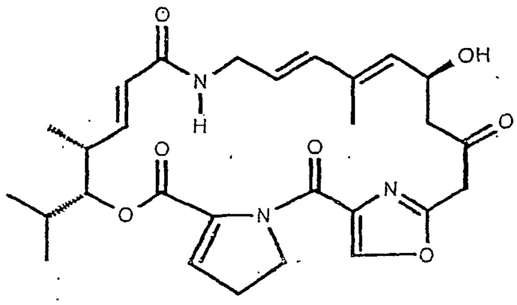

Pristinamycine $II_A$

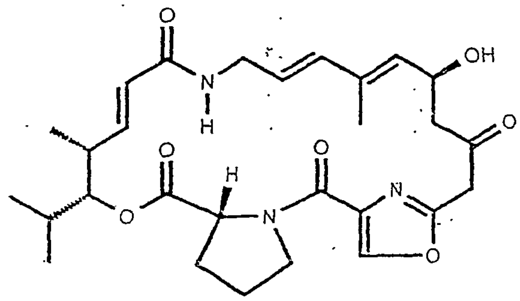

Pristinamycine $II_B$

Figure 1

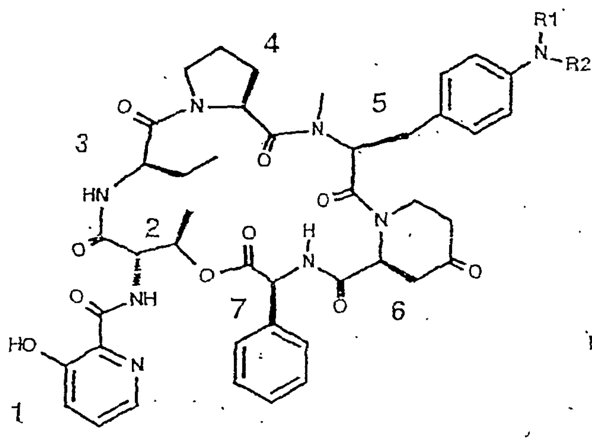

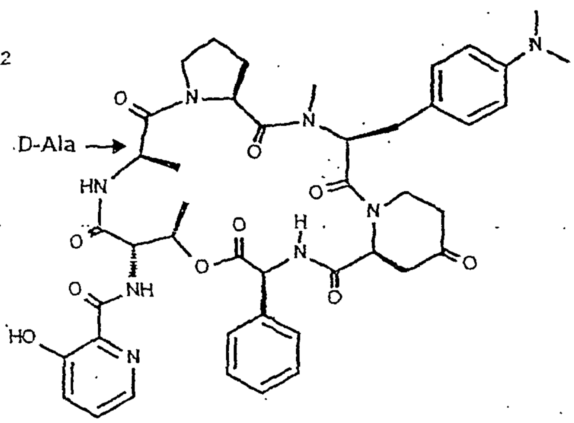

Pristinamycine $I_A$ R1 = R2 = Me

Pristinamycine $I_B$ R1 = H, R2 = Me

PINH2 R1 = R2 = H

Pristinamycine $I_C$

Figure 2

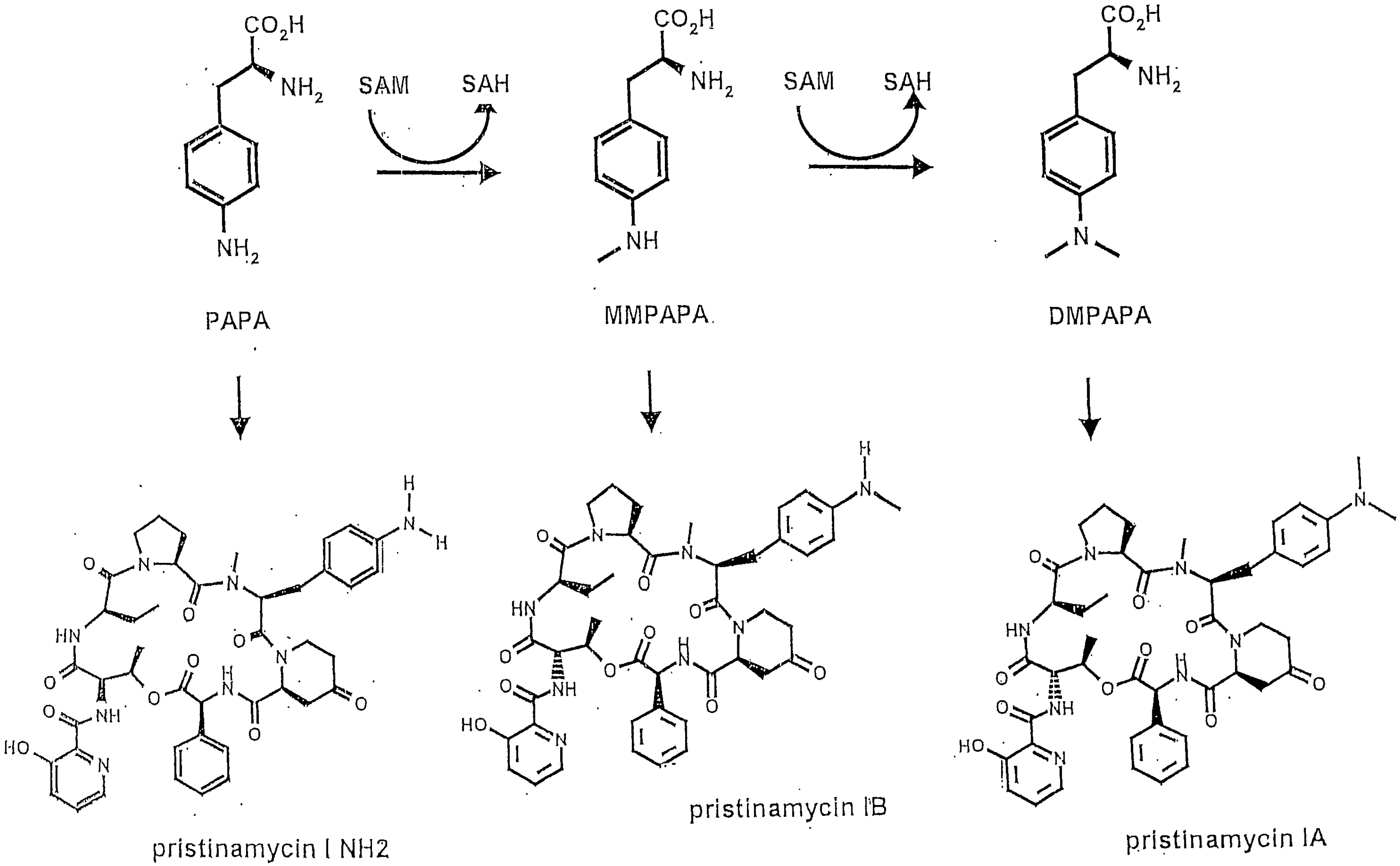


PAPA

MMPAPA

DMPAPA

pristinamycin I NH2

pristinamycin IB

pristinamycin IA

Figure 3

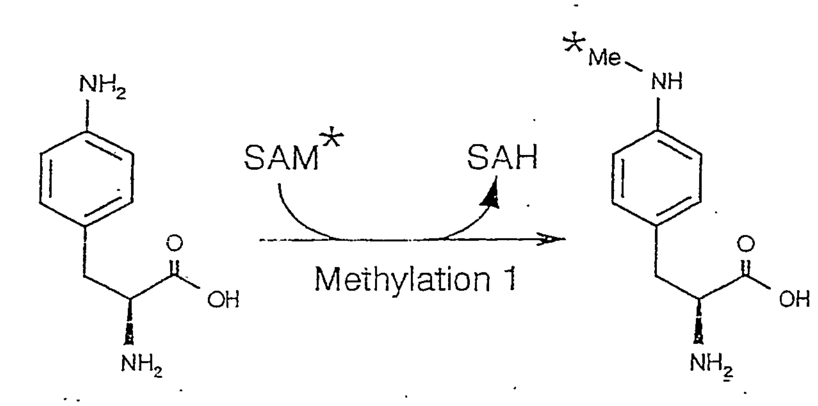


PAPA MMPAPA

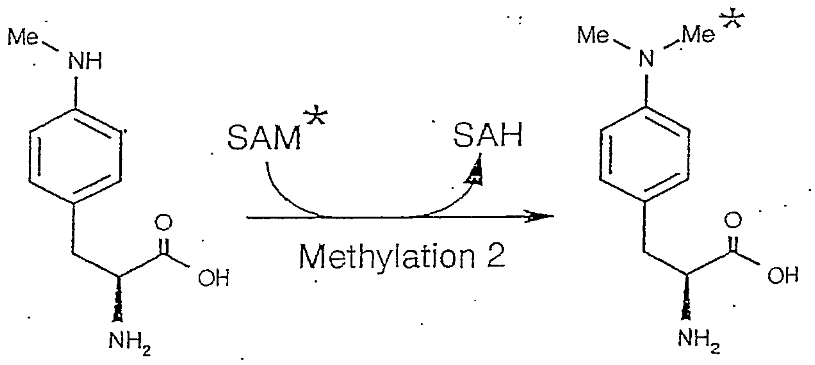


MMPAPA DMPAPA

Figure 4

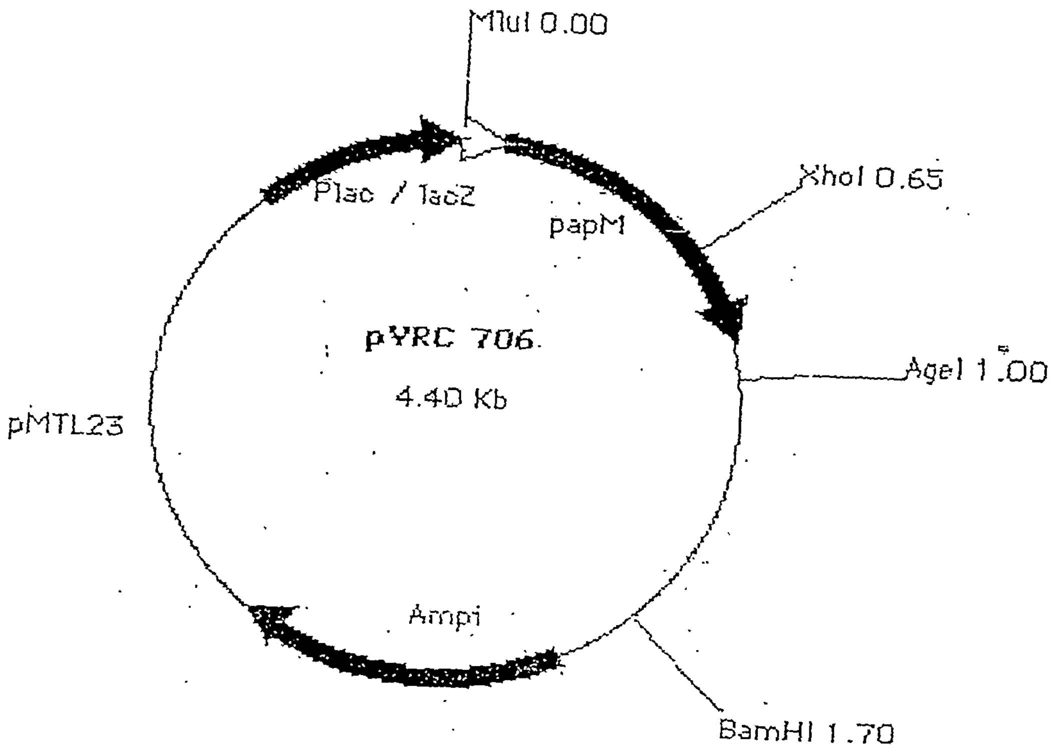
MluI 0.00
XhoI 0.65
AgeI 1.00
BamHI 1.70
Plac / lacZ
papM
pYRC 706
4.40 Kb
pMTL23
Ampi

Figure 5

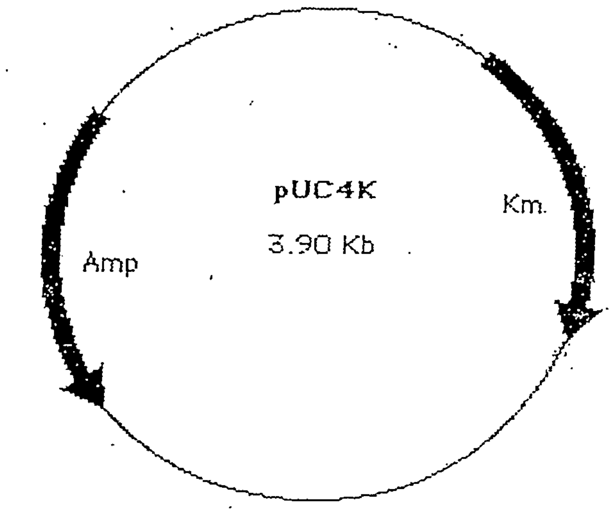


Figure 6

```
 M   T   A   A   A   P   T   L   A   Q   A   L   D   E   A   T   G   Q    18
GTG ACC GCC GCC GCA CCC ACC CTC GCC CAG GCG CTG GAC GAG GCC ACC GGG CAG   54

 L   T   G   A   G   I   T   A   D   A   A   R   A   D   T   R   L   L    36
CTG ACC GGC GCC GGG ATC ACC GCC GAC GCC GCC CGG GCC GAC ACC CGG CTG CTG  108

 A   A   H   A   C   Q   V   A   P   G   D   L   D   T   C   L   A   G    54
GCC GCC CAC GCC TGC CAG GTC GCC CCG GGG GAC CTC GAC ACC TGC CTG GCC GGC  162

 P   V   P   P   R   F   W   H   Y   V   R   R   R   L   T   R   E   P    72
CCG GTG CCG CCC CGG TTC TGG CAC TAC GTC CGG CGC CGT CTG ACC CGC GAA CCC  216

 A   E   R   I   V   G   H   A   Y   F   M   G   H   R   F   D   L   A    90
GCC GAA CGC ATC GTC GGC CAC GCC TAC TTC ATG GGC CAC CGC TTC GAC CTG GCC  270

 P   G   V   F   V   P   K   P   E   T   E   E   I   T   R   D   A   I   108
CCC GGC GTC TTC GTC CCC AAA CCC GAG ACC GAG GAG ATC ACC CGG GAC GCC ATC  324

 A   R   L   E   A   L   V   R   R   G   T   P   A   P   L   V   V   D   126
GCC CGC CTG GAG GCC CTC GTC CGC CGC GGC ACC CCC GCA CCC CTG GTC GTC GAC  378

 L   C   A   G   P   G   T   M   A   V   T   L   A   R   H   V   P   A   144
CTG TGC GCC GGA CCG GGC ACC ATG GCC GTC ACC CTG GCC CGC CAC GTA CCG GCC  432

 A   R   V   L   G   I   E   L   S   Q   A   A   A   R   A   A   R   R   162
GCC CGC GTC CTG GGC ATC GAA CTC TCC CAG GCC GCC GCC CGC GCC GCC CGG CGC  486

 N   A   R   G   T   G   A   R   I   V   Q   G   D   A   R   D   A   F   180
AAC GCC CGC GGC ACC GGC GCC CGC ATC GTG CAG GGC GAC GCC CGC GAC GCC TTC  540

                                             Thr>Ile
 P   E   L   S   G   T   V   D   L   V   V   T   N   P   P   Y   I   P   198
CCC GAA CTG AGC GGC ACC GTC GAC CTC GTC GTC ACC AAC CCG CCC TAC ATC CCC  594
                                             T           T   T       T
                                            <<<<<<<<mutant66>>>>>>>>>>>>

 I   G   L   R   T   S   A   P   E   V   L   E   H   D   P   P   L   A   216
ATC GGA CTG CGC ACC TCC GCA CCC GAA GTG CTC GAG CAC GAC CCG CCG CTG GCC  648

 L   W   A   G   E   E   G   L   G   M   I   R   A   M   E   R   T   A   234
CTG TGG GCC GGG GAG GAG GGC CTC GGC ATG ATC CGC GCC ATG GAA CGC ACC GCG  702

                                                     Gly>Ser
 A   R   L   L   A   P   G   G   V   L   L   L   E   H   G   S   Y   Q   252
GCC CGG CTG CTG GCC CCC GGC GGC GTC CTG CTC CTC GAA CAC GGC TCC TAC CAA  756
                                                         A
                                                     mutant49

 L   A   S   V   P   A   L   F   R   A   T   G   R   W   S   H   A   S   270
CTC GCC TCC GTG CCC GCC CTG TTC CGC GCA ACC GGC CGC TGG AGC CAC GCC TCG  810

 S   R   P   T   C   N   D   G   C   L   T   A   V   R   N   H   T   C   288
TCC CGT CCC ACC TGC AAC GAC GGC TGC CTG ACC GCC GTA CGC AAC CAC ACC TGC  864

 A   P   P   A   *                                                       293
GCA CCG CCC GCC TGA                                                      879
```

Figure 7

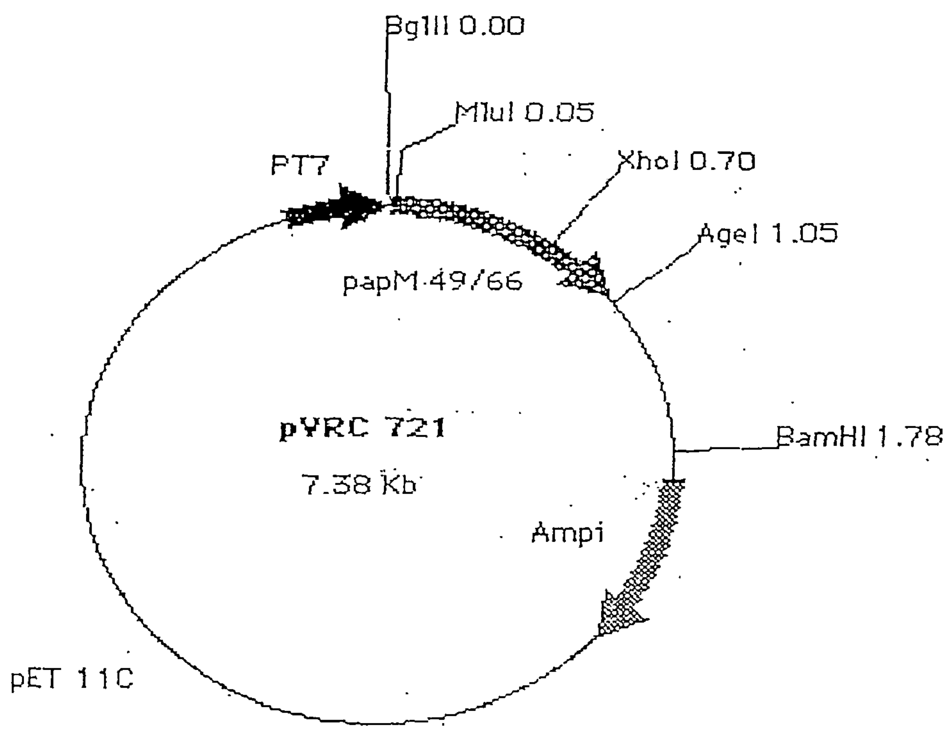
BglII 0.00
MluI 0.05
PT7
XhoI 0.70
AgeI 1.05
papM-49/66
pYRC 721
7.38 Kb
BamHI 1.78
Ampi
pET 11C

Figure 8

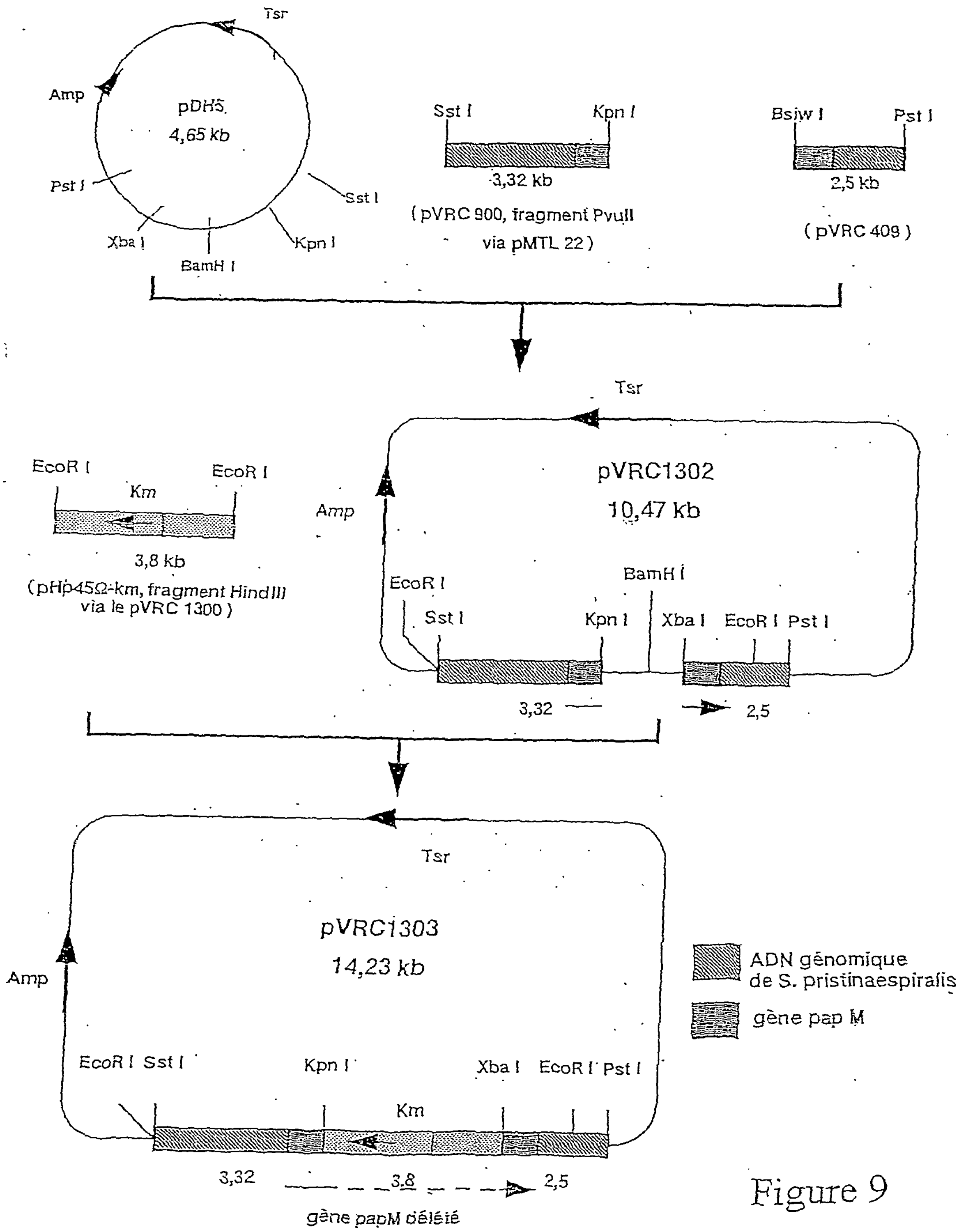

Tsr
Amp
pDH5
4,65 kb
Pst I
Sst I
Xba I
Kpn I
BamH I
Sst I
Kpn I
3,32 kb
(pVRC 900, fragment PvuII via pMTL 22)
BsiW I
Pst I
2,5 kb
(pVRC 409)
Tsr
EcoR I
EcoR I
Km
3,8 kb
(pHp45Ω-km, fragment HindIII via le pVRC 1300)
Amp
pVRC1302
10,47 kb
EcoR I
BamH I
Sst I
Kpn I
Xba I
EcoR I
Pst I
3,32
2,5
Tsr
pVRC1303
14,23 kb
Amp
EcoR I
Sst I
Kpn I
Xba I
EcoR I
Pst I
Km
3,32
3,8
2,5
gène papM délété
ADN génomique de S. pristinaespiralis
gène pap M


Figure 9

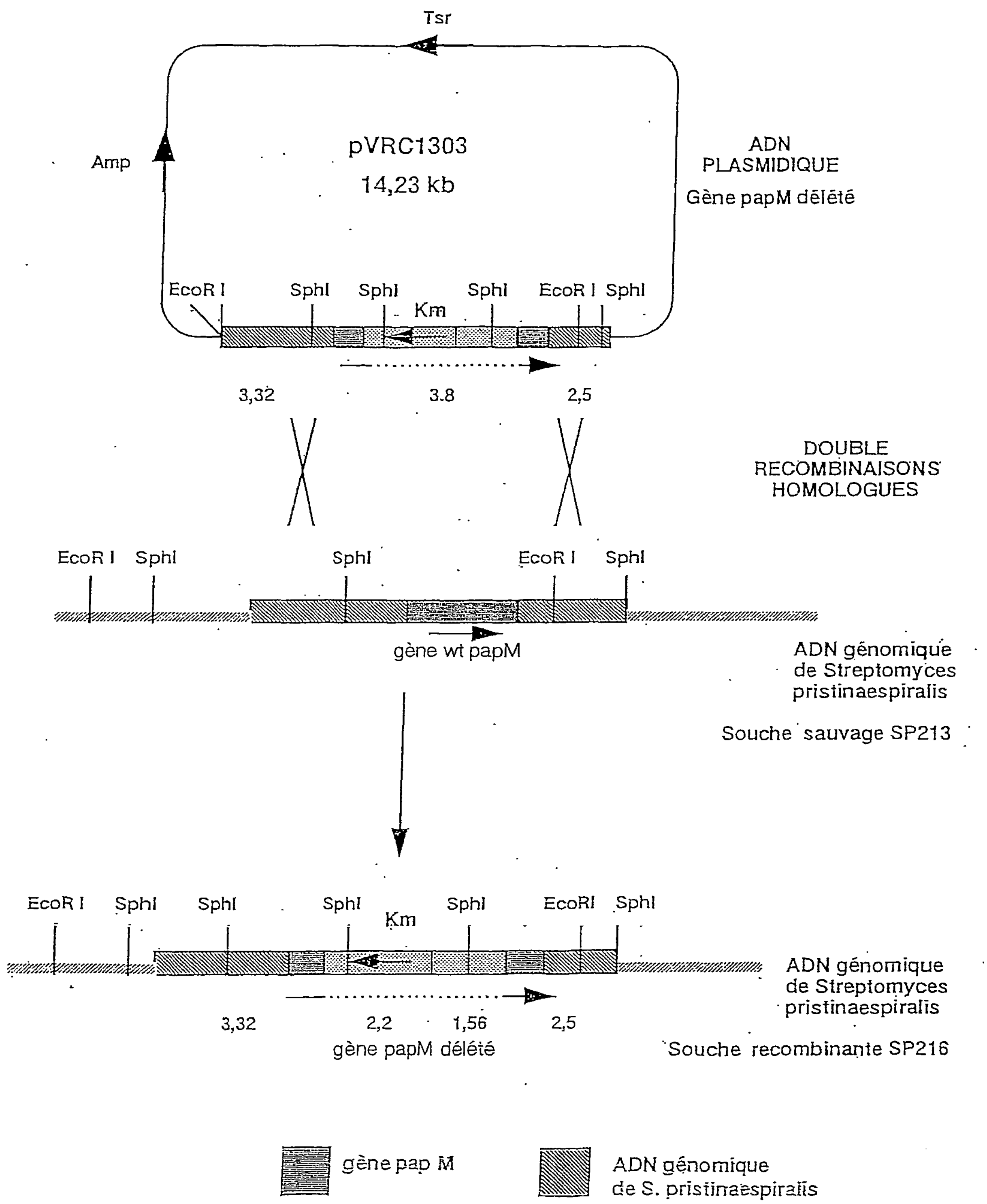
Tsr
pVRC1303
14,23 kb
Amp
ADN
PLASMIDIQUE
Gène papM délété
EcoR I
SphI
SphI
SphI
EcoR I
SphI
Km
3,32
3.8
2,5
DOUBLE
RECOMBINAISONS
HOMOLOGUES
EcoR I
SphI
SphI
EcoR I
SphI
gène wt papM
ADN génomique
de Streptomyces
pristinaespiralis
Souche sauvage SP213
EcoR I
SphI
SphI
SphI
Km
SphI
EcoRI
SphI
ADN génomique
de Streptomyces
pristinaespiralis
3,32
2,2
1,56
2,5
gène papM délété
Souche recombinante SP216
gène pap M
ADN génomique
de S. pristinaespiralis

Figure 10

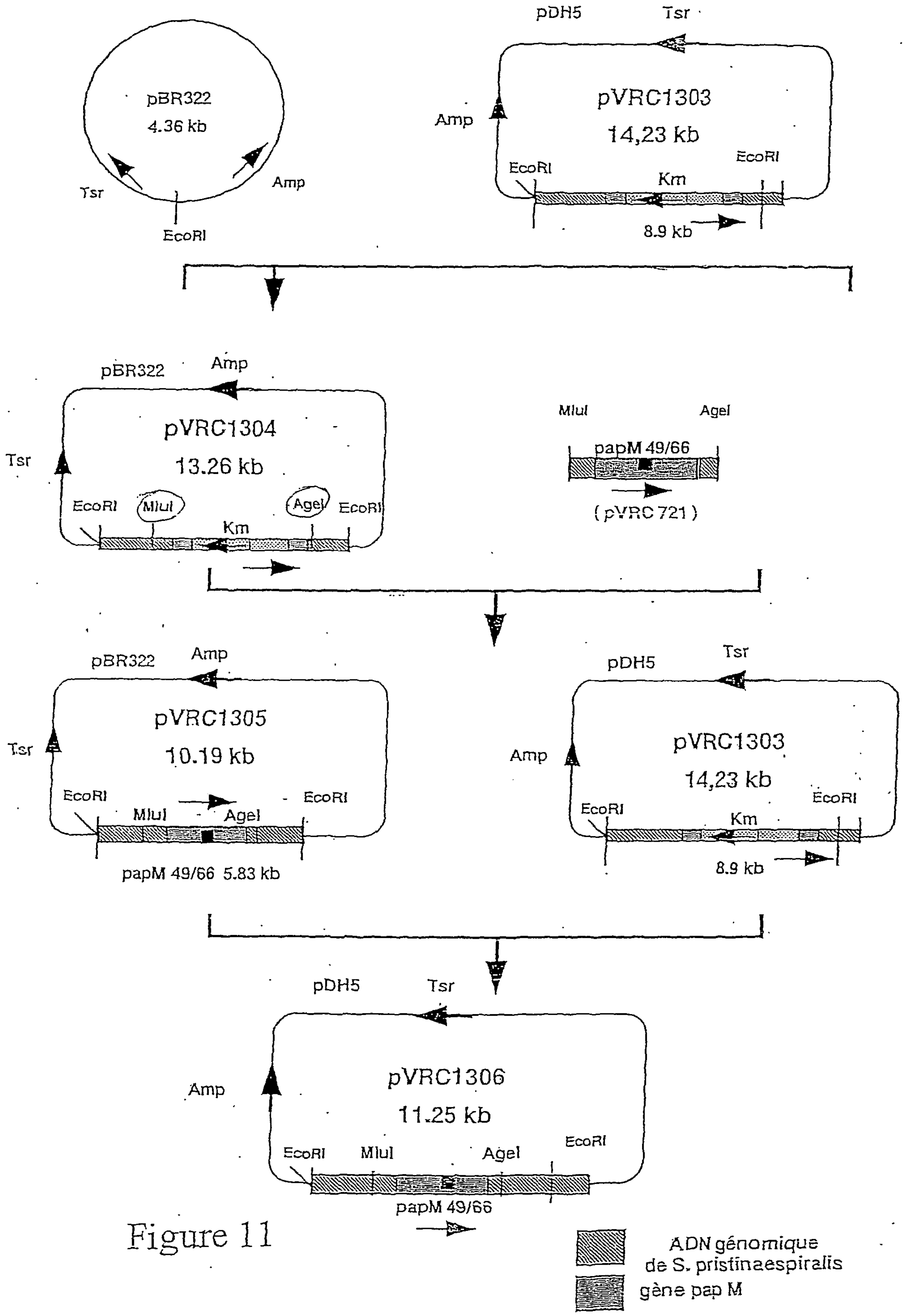
pBR322
4.36 kb
Tsr
Amp
EcoRI
pDH5
Tsr
pVRC1303
14,23 kb
Amp
EcoRI
Km
EcoRI
8.9 kb
pBR322
Amp
pVRC1304
13.26 kb
Tsr
EcoRI
MluI
AgeI
EcoRI
Km
MluI
AgeI
papM 49/66
(pVRC 721)
pBR322
Amp
pVRC1305
10.19 kb
Tsr
EcoRI
MluI
AgeI
EcoRI
papM 49/66 5.83 kb
pDH5
Tsr
pVRC1303
14,23 kb
Amp
EcoRI
Km
EcoRI
8.9 kb
pDH5
Tsr
pVRC1306
11.25 kb
Amp
EcoRI
MluI
AgeI
EcoRI
papM 49/66
ADN génomique de S. pristinaespiralis
gène pap M

Figure 11

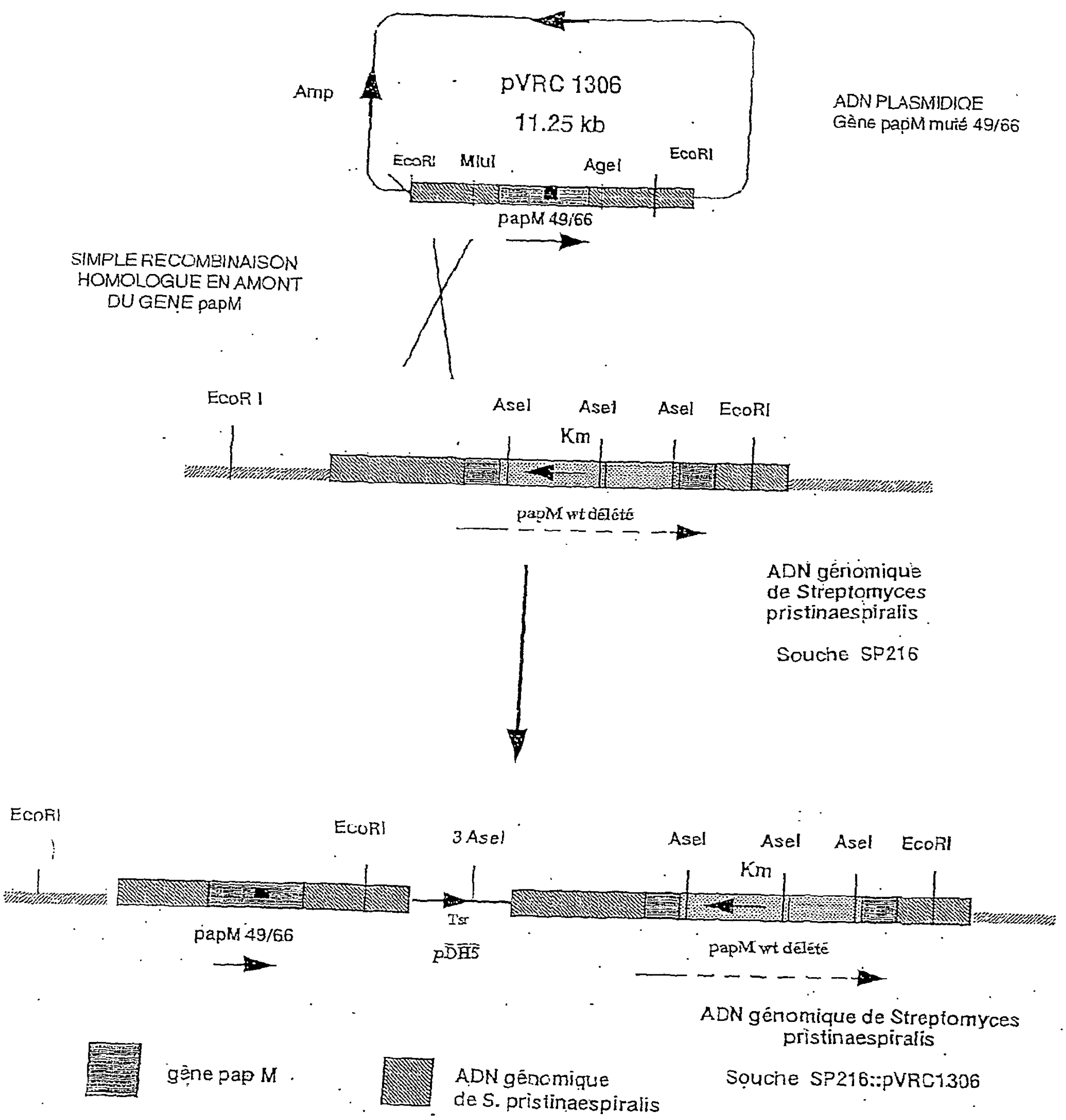
Amp
pVRC 1306
11.25 kb
ADN PLASMIDIQE
Gène papM muté 49/66
EcoRI
MluI
AgeI
EcoRI
papM 49/66
SIMPLE RECOMBINAISON
HOMOLOGUE EN AMONT
DU GENE papM
EcoR I
AseI
AseI
AseI
EcoRI
Km
papM wt délété
ADN génomique
de Streptomyces
pristinaespiralis
Souche SP216
EcoRI
EcoRI
3 AseI
AseI
AseI
AseI
EcoRI
Km
Tsr
papM 49/66
pDH5
papM wt délété
ADN génomique de Streptomyces
pristinaespiralis
gène pap M
ADN génomique
de S. pristinaespiralis
Souche SP216::pVRC1306

Figure 12

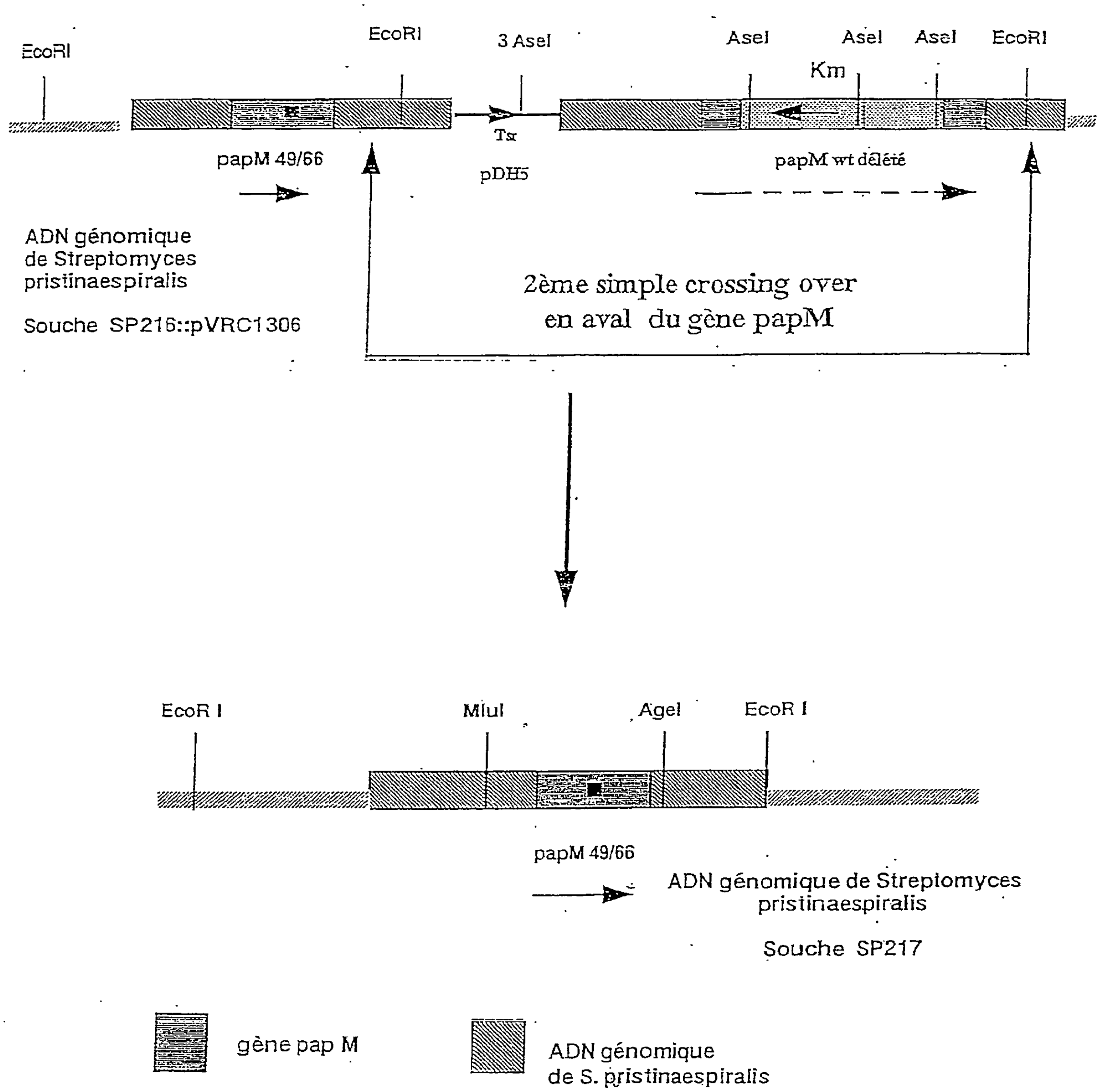
EcoRI
EcoRI
3 AseI
AseI
AseI
AseI
EcoRI
Km
Tx
papM 49/66
pDH5
papM wt délété
ADN génomique
de Streptomyces
pristinaespiralis
Souche SP216::pVRC1306
2ème simple crossing over
en aval du gène papM
EcoR I
MluI
AgeI
EcoR I
papM 49/66
ADN génomique de Streptomyces
pristinaespiralis
Souche SP217
gène pap M
ADN génomique
de S. pristinaespiralis

Figure 13

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Documents brevets cités dans la description


- WO 9605219 A
- WO 9601901 A
- WO 9320182 A
- WO 9408014 A
- WO 9601901 A1
- FR 0208057


### Littérature non-brevet citée dans la description


- **Cocito C. G.** *Microbiol. Rev.,* 1979, vol. 43, 145-198
- **Cocito C. G.** In Antibiotics. 1983, vol. 6, 296-332
- **Di Giambattista M. ; Chinali G. ; Cocito C. G.** *J. Antim. Chemother.,* 1989, vol. 24, 485-507
- **Blanc et al.** *J. Bacteriol.,* 1995, vol. 177 (18), 5206-14
- **Thibaut et al.** *J. Bacteriol.,* 1997, vol. 179 (3), 697-704
- **Blanc et al.** *Mol. Microbiol,* 1997, vol. 23 (2), 191-202
- **Chambers et al.** *Gene,* 15 Août 1988, vol. 68 (1), 139-49
- **Cocito C. G.** Antibiotics. 1983, vol. 6, 296-332
- **Dessen P. C. ; Fondrat C. ; Valencien C. ; Mugnier C.** *Comp. Appl. in Biosciences,* 1990, vol. 6, 355-356
- **Fellay et al.** *Gene,* 1987, vol. 52 (2-3), 147-54
- **Hanahan et al.** *PNAS USA,* 1990, vol. 87, 4645-4649
- **Hillemann D. ; Pülher A. ; Wohlleben W.** *Nucl. Acids Res.,* 1991, vol. 19, 727-731
- **Hopwood D. A. ; Bibb M. J. ; Chater K. F. ; Kieser T. ; Bruton C. J. ; Kieser H. M. ; Lydiate D. J. ; Smith C. P. ; Ward J. M. ; Scrempf H.** A laboratory manual. The John Innes Fondation, 1985
- **Humphreys et al.** *Mol Gen Genet.,* 23 Avril 1976, vol. 145 (1), 101-8
- **Maniatis T. ; Fritsh E. F. ; Sambrook J.** Molecular cloning : a laboratory manual. Cold Spring Harbor, 1989
- **Messing J. ; Crea R. ; Seeburg P. H.** *Nucleic Acids Res.,* 1981, vol. 9, 309
- **Schluckebier et al.** *Gene,* 19 Mai 1995, vol. 157 (1-2), 131-4
- **Veira ; Messing.** *Gene,* Octobre 1982, vol. 19 (3), 259-68

LISTE DE SEQUENCES

<110> AVENTIS PHARMA S.A.

<120> Nouveaux variants du polypeptide papM de bactéries du genre Streptomyces

<130> Polypeptide PapM

<140> FR0208057
<141> 2002-06-28

<160> 2

<170> PatentIn Ver. 2.1

<210> 1
<211> 1052
<212> ADN
<213> Streptomyces pristinaespiralis

<220>
<221> CDS
<222> (84)..(962)

<400> 1

ctcgaggacg agtggatcgc ctccggcggc gcccccgtcc ccacgcccgt gcacgcgtcc 60

gcgtccgcgc ggggggccgt gtc gtg acc gcc gcc gca ccc acc ctc gcc cag 113
Val Thr Ala Ala Ala Pro Thr Leu Ala Gln
1 5 10

gcg ctg gac gag gcc acc ggg cag ctg acc ggc gcc ggg atc acc gcc 161
Ala Leu Asp Glu Ala Thr Gly Gln Leu Thr Gly Ala Gly Ile Thr Ala
15 20 25

gac gcc gcc cgg gcc gac acc cgg ctg ctg gcc gcc cac gcc tgc cag 209
Asp Ala Ala Arg Ala Asp Thr Arg Leu Leu Ala Ala His Ala Cys Gln
30 35 40

gtc gcc ccg ggg gac ctc gac acc tgc ctg gcc ggc ccg gtg ccg ccc 257
Val Ala Pro Gly Asp Leu Asp Thr Cys Leu Ala Gly Pro Val Pro Pro
45 50 55

cgg ttc tgg cac tac gtc cgg cgc cgt ctg acc cgc gaa ccc gcc gaa 305

Arg Phe Trp His Tyr Val Arg Arg Arg Leu Thr Arg Glu Pro Ala Glu
60 65 70

cgc atc gtc ggc cac gcc tac ttc atg ggc cac cgc ttc gac ctg gcc 353
Arg Ile Val Gly His Ala Tyr Phe Met Gly His Arg Phe Asp Leu Ala
75 80 85 90

ccc ggc gtc ttc gtc ccc aaa ccc gag acc gag gag atc acc cgg gac 401
Pro Gly Val Phe Val Pro Lys Pro Glu Thr Glu Glu Ile Thr Arg Asp
95 100 105

gcc atc gcc cgc ctg gag gcc ctc gtc cgc cgc ggc acc acc gca ccc 449
Ala Ile Ala Arg Leu Glu Ala Leu Val Arg Arg Gly Thr Thr Ala Pro
110 115 120

ctg gtc gtc gac ctg tgc gcc gga ccg ggc acc atg gcc gtc acc ctg 497
Leu Val Val Asp Leu Cys Ala Gly Pro Gly Thr Met Ala Val Thr Leu
125 130 135

gcc cgc cac gta ccg gcc gcc cgc gtc ctg ggc atc gaa ctc tcc cag 545
Ala Arg His Val Pro Ala Ala Arg Val Leu Gly Ile Glu Leu Ser Gln
140 145 150

gcc gcc gcc cgc gcc gcc cgg cgc aac gcc cgc ggc acc ggc gcc cgc 593
Ala Ala Ala Arg Ala Ala Arg Arg Asn Ala Arg Gly Thr Gly Ala Arg
155 160 165 170

atc gtg cag ggc gac gcc cgc gac gcc ttc ccc gaa ctg agc ggc acc 641
Ile Val Gln Gly Asp Ala Arg Asp Ala Phe Pro Glu Leu Ser Gly Thr
175 180 185

gtc gac ctc gtc gtc acc aac ccg ccc tac atc ccc atc gga ctg cgc 689
Val Asp Leu Val Val Thr Asn Pro Pro Tyr Ile Pro Ile Gly Leu Arg
190 195 200

acc tcc gca ccc gaa gtg ctc gag cac gac ccg ccg ctg gcc ctg tgg 737
Thr Ser Ala Pro Glu Val Leu Glu His Asp Pro Pro Leu Ala Leu Trp
205 210 215

gcc ggg gag gag ggc ctc ggc atg atc cgc gcc atg gaa cgc acc gcg 785
Ala Gly Glu Glu Gly Leu Gly Met Ile Arg Ala Met Glu Arg Thr Ala
220 225 230

gcc cgg ctg ctg gcc ccc ggc ggc gtc ctg ctc ctc gaa cac ggc tcc 833
Ala Arg Leu Leu Ala Pro Gly Gly Val Leu Leu Leu Glu His Gly Ser

235 240 245 250

tac caa ctc gcc tcc gtg ccc gcc ctg ttc cgc gca acc ggc cgc tgg 881
Tyr Gln Leu Ala Ser Val Pro Ala Leu Phe Arg Ala Thr Gly Arg Trp
255 260 265

agc cac gcc tcg tcc cgt ccc acc tgc aac gac ggc tgc ctg acc gcc 929
Ser His Ala Ser Ser Arg Pro Thr Cys Asn Asp Gly Cys Leu Thr Ala
270 275 280

gta cgc aac cac acc tgc gca ccg ccc gcc tga cacggcgtca cggcacggcc 982
Val Arg Asn His Thr Cys Ala Pro Pro Ala
285 290

ggcctgtcgg caacgaccct acgccattga caaaccgacc gtgccgtttt tttaatgtcg 1042

gggtggcgga 1052

<210> 2
<211> 292
<212> PRT
<213> Streptomyces pristinaespiralis

<400> 2

Val Thr Ala Ala Ala Pro Thr Leu Ala Gln Ala Leu Asp Glu Ala Thr
1 5 10 15

Gly Gln Leu Thr Gly Ala Gly Ile Thr Ala Asp Ala Ala Arg Ala Asp
20 25 30

Thr Arg Leu Leu Ala Ala His Ala Cys Gln Val Ala Pro Gly Asp Leu
35 40 45

Asp Thr Cys Leu Ala Gly Pro Val Pro Pro Arg Phe Trp His Tyr Val
50 55 60

Arg Arg Arg Leu Thr Arg Glu Pro Ala Glu Arg Ile Val Gly His Ala
65 70 75 80

Tyr Phe Met Gly His Arg Phe Asp Leu Ala Pro Gly Val Phe Val Pro
85 90 95

Lys Pro Glu Thr Glu Glu Ile Thr Arg Asp Ala Ile Ala Arg Leu Glu
100 105 110

Ala Leu Val Arg Arg Gly Thr Thr Ala Pro Leu Val Val Asp Leu Cys
115 120 125

Ala Gly Pro Gly Thr Met Ala Val Thr Leu Ala Arg His Val Pro Ala
130 135 140

Ala Arg Val Leu Gly Ile Glu Leu Ser Gln Ala Ala Ala Arg Ala Ala
145 150 155 160

Arg Arg Asn Ala Arg Gly Thr Gly Ala Arg Ile Val Gln Gly Asp Ala
165 170 175

Arg Asp Ala Phe Pro Glu Leu Ser Gly Thr Val Asp Leu Val Val Thr
180 185 190

Asn Pro Pro Tyr Ile Pro Ile Gly Leu Arg Thr Ser Ala Pro Glu Val
195 200 205

Leu Glu His Asp Pro Pro Leu Ala Leu Trp Ala Gly Glu Glu Gly Leu
210 215 220

Gly Met Ile Arg Ala Met Glu Arg Thr Ala Ala Arg Leu Leu Ala Pro
225 230 235 240

Gly Gly Val Leu Leu Leu Glu His Gly Ser Tyr Gln Leu Ala Ser Val
245 250 255

Pro Ala Leu Phe Arg Ala Thr Gly Arg Trp Ser His Ala Ser Ser Arg
260 265 270

Pro Thr Cys Asn Asp Gly Cys Leu Thr Ala Val Arg Asn His Thr Cys
275 280 285

Ala Pro Pro Ala
290